# EUROPEAN PATENT APPLICATION

(11) **EP 2 541 897 A2**
(43) Date of publication of application: **02.01.2013**
(21) Application number: 12174423.9
(22) Date of filing: 29.06.2012
(51) Int. Cl.: H04N 5/3745, H04N 5/376

(54) **Imaging apparatus, imaging system, control apparatus, and method for controlling image sensor**

(30) Priority: 29.06.2011 JP 2011144070
(71) Applicant: CANON KABUSHIKI KAISHA, Ohta-ku Tokyo 146-8501 (JP)
(72) Inventor: Dowaki, Kanako, Ohta-ku, Tokyo (JP); Matsumoto, Kazumasa, Ohta-ku, Tokyo (JP)
(74) Representative: Northway, Daniel Robert

(57) **Abstract**

An imaging apparatus (101) includes an image sensor (111) having an imaging area with a plurality of pixels arranged therein, each pixel having a photoelectric conversion means and pixel amplifiers, a control means (100) for performing control for shifting the timing of activating the pixel amplifiers for each of sub areas in the imaging area, and control for reading an electrical signal from each pixel acquired via the activated pixel amplifiers, and a generation means for generating an image based on the read electrical signal.

## Description

### BACKGROUND OF THE INVENTION

### Field of the Invention

The present invention relates to an imaging apparatus, an imaging system, a control apparatus, and a method for controlling an image sensor.

### Description of the Related Art

In recent years, imaging apparatuses for generating digital images have been widely used in medical field, other industrial fields, and consumer products. For example, in medical field, an increasing number of large area flat-panel type image sensors for same-size optical systems using photoelectric conversion elements are being used. Image sensors of this type are more advantageous than conventional image intensifiers in terms of improved resolution, reduced volume, and reduced image distortion.

By providing amplifiers, such as source follower amplifiers, in a pixel of such an image sensor, the signal-to-noise (S/N) ratio can be improved. Further, by controlling such amplifiers (pixel amplifiers) to operate in case of capturing an electrical signal from a photoelectric conversion element and not to operate in other cases, power consumption by the pixel amplifiers can be reduced. However, there has been a case where activating at one time the pixel amplifiers of pixels in the image sensor generates a rush current which adversely affects an image output by the image sensor.

### SUMMARY OF THE INVENTION

The present invention in its first aspect provides an imaging apparatus as specified in claims 1 to 19.

Further features and aspects of the present invention will become apparent from the following detailed description of exemplary embodiments with reference to the attached drawings.

### BRIEF DESCRIPTION OF THE DRAWINGS

The accompanying drawings, which are incorporated in and constitute a part of the specification, illustrate exemplary embodiments, features, and aspects of the invention and, together with the description, serve to explain the principles of the invention.

Fig. 1 illustrates an overall configuration of an X-ray imaging system according to a first exemplary embodiment of the present invention.

Fig. 2 illustrates a configuration of an imaging apparatus.

Fig. 3 illustrates a configuration of an image sensor.

Fig. 4 illustrates an overview of an equivalent circuit of a pixel.

Fig. 5 is a flowchart illustrating an operation flow of the imaging apparatus and a control apparatus.

Fig. 6A is a timing chart illustrating an imaging operation of the image sensor, and Fig. 6B is a flowchart illustrating the activation of a pixel amplifier and a sample-hold circuit.

Fig. 7A is a graph illustrating temporal voltage change according to the first exemplary embodiment, and Fig. 7B is a graph illustrating temporal voltage change according to a comparative example.

Fig. 8A is a timing chart illustrating driving of a pixel amplifier and the sample-hold circuit according to the comparative example, and Fig. 8B is timing chart illustrating driving of the pixel amplifier and the sample-hold circuit according to other comparative examples.

Fig. 9 illustrates a configuration of an imaging apparatus according to a second exemplary embodiment.

Fig. 10 is a flowchart illustrating an operation flow of the imaging apparatus according to the second exemplary embodiment.

Fig. 11A is a graph illustrating temporal voltage change according to the second exemplary embodiment, and Fig. 11B is a graph illustrating temporal current change according to the second exemplary embodiment.

Fig. 12 illustrates a configuration of an imaging apparatus according to a third exemplary embodiment.

Fig. 13 is a flowchart illustrating an operation flow of the imaging apparatus according to the third exemplary embodiment.

Figs. 14A and 14B are timing charts illustrating imaging operations of an image sensor according to the third exemplary embodiment.

Fig. 15 illustrates a configuration of an imaging apparatus according to a fourth exemplary embodiment.

Fig. 16 is a flowchart illustrating an operation flow of the imaging apparatus according to the fourth exemplary embodiment.

Fig. 17 illustrates a hardware configuration of a control apparatus according to the first exemplary embodiment.

### DESCRIPTION OF THE EMBODIMENTS

Various exemplary embodiments, features, and aspects of the invention will be described in detail below with reference to the drawings.

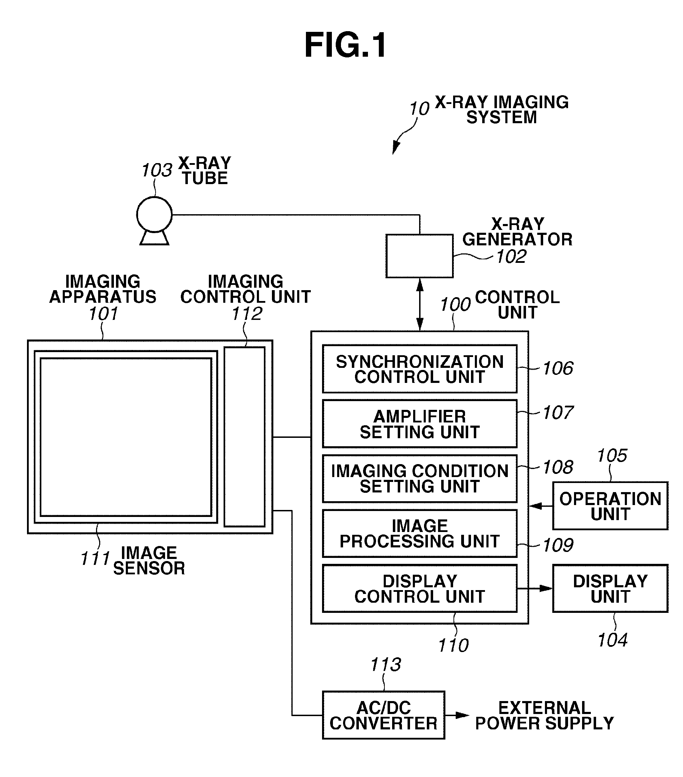

An X-ray imaging system according to a first exemplary embodiment will be described below with reference to Figs. 1 to 8. An overall configuration of an X-ray imaging system 10 will be described below with reference to Fig. 1.

The X-ray imaging system 10 includes a control apparatus 100, an imaging apparatus 101, an X-ray generator 102, an X-ray tube 103, a display unit 104, an operation unit 105, and an AC/DC converter 113. The X-ray imaging system 10 is used in, for example, a visiting car, C arm, and computerized tomography (CT) imaging system.

The control apparatus 100 controls the entire X-ray imaging system. The control apparatus 100 includes a synchronization control unit 106, an amplifier setting unit 107, an imaging condition setting unit 108, an image processing unit 109, and a display control unit 110. The imaging condition setting unit 108 gives instructions to the imaging apparatus 101 and the X-ray generator 102 about imaging conditions based on order inputs from the operation unit 105 or the outside.

The synchronization control unit 106 performs synchronization control for synchronizing the X-ray irradiation by the X-ray tube 103, with the X-ray reception and reading by the imaging apparatus 101 in response to the depression of an X-ray irradiation switch (not illustrated). The synchronization control unit 106 performs synchronization control by transmitting a pulse signal to the imaging apparatus 101 and the X-ray generator 102. With synchronization control, the X-ray generator 102 generates X-ray from the X-ray tube 103 at predetermined timing. The imaging apparatus 101 captures an image of the generated X-ray and then transmits the captured image to the control apparatus 100.

The image processing unit 109 performs predetermined processing on the captured image, and then the display control unit 110 displays the processed image on the display unit 104. Under control of the control apparatus 100, the imaging apparatus 101 repeats image output processing a plurality of times in succession to capture a moving image, and performs single-shot imaging to capture a still image.

The amplifier setting unit 107 makes settings for controlling the activation timing of pixel amplifiers included in an image sensor of the imaging apparatus 101. Specifically, the amplifier setting unit 107 makes settings to drive the pixel amplifiers in case of transmitting an electrical signal produced in the photoelectric conversion element to a sample-hold circuit, and not to drive the pixel amplifiers in other cases. Further, the amplifier setting unit 107 separately sets the pixel amplifier activation timing for each sub area of the image sensor. Settings for pixel amplifier control are made based on at least one of input from the operation unit 105, information reception from an external device, setting information stored in a memory (not illustrated) in the control apparatus 100. The pixel amplifier control will be described below.

The imaging apparatus 101 mainly includes an image sensor 111 and an imaging control unit 112 for controlling the image sensor 111. The imaging apparatus 101 is, for example, a flat panel detector or X-ray detector used for X-ray imaging. The imaging apparatus 101 converts X-ray into visible light with phosphor (not illustrated) and the image sensor 111 receives the visible light to generate charge carriers. Thus, the imaging apparatus 101 acquires an X-ray image according to the dose distribution of X-ray that has reached the imaging apparatus 101.

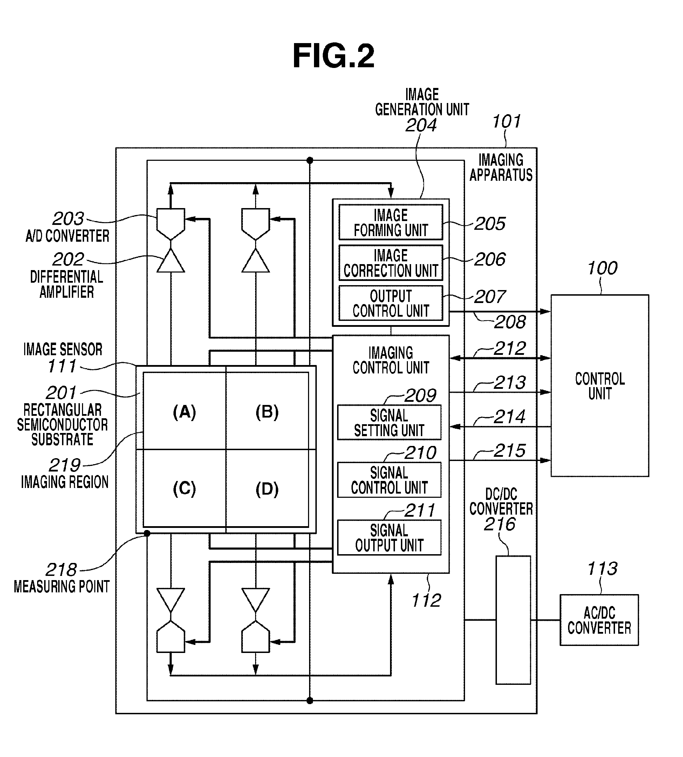

A configuration of the imaging apparatus 101 will be described in detail below with reference to Fig. 2. The imaging apparatus 101 includes the image sensor 111, differential amplifiers 202, A/D converters 203, an image generation unit 204, the imaging control unit 112, and a DC/DC converter 216.

The image sensor 111 is formed of a plurality of CMOS rectangular semiconductor substrates 201 bonded together. The image sensor 111 includes a plurality of two-dimensionally arranged pixels. The two-dimensionally arranged pixels form an imaging area 219. When the imaging area 219 is irradiated with X-ray, the pixels of the image sensor 111 receive light to produce an analog electric signal.

A differential amplifier 202 amplifies the analog electric signal read from the image sensor 111, and an A/D converter 203 converts the analog signal into a digital signal.

The image generation unit 204 generates captured image data from the acquired digital signal. An image forming unit 205 of the image generation unit 204 arranges digital signals to form image data. An image correction unit 206 performs dark correction processing in which dark image data accumulated without X-ray reception is subtracted from image data acquired by the image sensor 111 receiving X-ray. An output control unit 207 outputs the captured image to the control apparatus 100 via an image data interface 208.

The imaging control unit 112 make settings for signals to be output to the image sensor 111, and outputs a driving signal for the image sensor 111 at suitable timing. A signal setting unit 209 of the imaging control unit 112 sets signals based on imaging conditions received from the control apparatus 100 via a command control interface 212. One of set signals is the activation interval of the pixel amplifiers (hereinafter referred to as pixel amplifier activation interval). The imaging control unit 112 transmits to the control apparatus 100 a radiation imaging apparatus state, such as imaging mode setting, various parameter settings, imaging start setting, and imaging end setting, via the command control interface 212. The signal control unit 210 controls the timing of outputting the driving signal to be output from the signal output unit 211 while monitoring the driving state of the image sensor 111 and synchronization signals from the control apparatus 100.

The imaging control unit 112 transmits a READY signal 213, one of synchronization signals, to the control apparatus 100 to notify that the image sensor 111 of the imaging apparatus 101 has become ready for accumulating charge carriers. Upon reception of the READY signal 213 from the imaging control unit 112, the control apparatus 100 transmits an external synchronization signal 214 to the imaging control unit 112 to notify the X-ray exposure timing. While an exposure permission signal 215 is enabled, the control apparatus 100 transmits an exposure signal to the X-ray generator 102. In response to the exposure signal, the X-ray tube 103 emits X-ray. Meanwhile, in response to the driving signal output from the signal control unit 210, the image sensor 111 outputs an electrical signal upon reception of X-ray to provide digital data of a captured image.

The DC/DC converter 216 converts a direct current (DC) voltage sent from the AC/DC converter 113, and supplies the resultant DC voltage in the imaging apparatus 101.

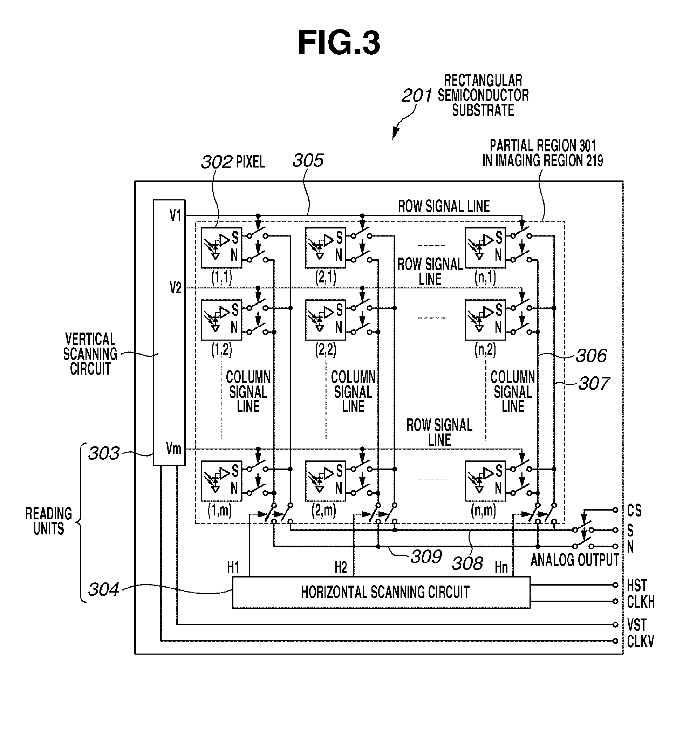

A configuration of the rectangular semiconductor substrates 201 constituting the image sensor 111 will be described below with reference to Fig. 3. The rectangular semiconductor substrates 201A to 201D are CMOS imaging devices formed of two-dimensional photoelectric conversion elements cut out from a silicon semiconductor wafer in rectangular form. The rectangular semiconductor substrates 201A to 201D are tiled on a planar base plate (not illustrated) in 2x2 matrix form. An imaging area on the rectangular semiconductor substrates 201A to 201D where pixels 302 exist is a sub area 301 of the imaging area 219.

Each of the rectangular semiconductor substrates 201 (also referred to as a rectangular semiconductor substrate 201) includes a plurality of pixels 302 arranged in the imaging area 219, a vertical scanning circuit 303, and a horizontal scanning circuit 304. The two scanning circuits 303 and 304 form an analog signal reading unit. The rectangular semiconductor substrate 201 further includes row signal lines 305 for transmitting a signal for selecting the pixels 302 for each row (generated by the vertical scanning circuit 303), column signal lines 306 and 307 for transmitting electrical signals read from the pixels 302, and analog output lines 308 and 309.

The rectangular semiconductor substrate 201 further includes a chip select signal input terminal CS, a light signal output terminal S, and a noise signal output terminal N which are collectively referred to as control terminals. The rectangular semiconductor substrate 201 further includes a vertical scanning circuit start signal terminal VST, a vertical scanning circuit clock terminal CLKV, a horizontal scanning circuit start signal terminal HST, and a horizontal scanning circuit clock terminal CLKH.

The vertical scanning circuit 303 selects a pixel group in the horizontal direction, and sequentially scans the pixel group in the vertical direction (sub scanning direction) in synchronization with a vertical scanning clock input from the vertical scanning circuit clock terminal CLKV. The horizontal scanning circuit 304 sequentially selects for each pixel in sequence the column signal lines 306 and 307 of the pixel group in the horizontal direction (main scanning direction) selected by the vertical scanning circuit 303 in synchronization with a horizontal scanning clock input from the horizontal scanning circuit clock terminal CLKH.

When a row signal line 305 (output line of the vertical scanning circuit 303) is enabled, relevant pixels 302 output a sample-held light signal voltage signal and a noise voltage signal to the column signal lines 307and 306, respectively.

Each of the row signal lines 305 is a signal transmission line for transmitting a signal for selecting pixels 302 for each row. A plurality of the row signal lines 305 is arranged in parallel with row lines. The column signal lines 306 and 307 are signal transmission lines for reading signals of selected pixels. A plurality of sets of the columns signal lines 306 and 306 is arranged in parallel with columns.

As the horizontal scanning circuit 304 sequentially selects voltage signals output to the column signal lines 306 and 307, voltage signals of each pixel are sequentially output to the analog output signal lines 308 and 309. The analog output signal lines 308 and 309 output the signals of the column signal line 307 and 306 to the A/D converter, respectively.

As described above, the rectangular semiconductor substrate 201 performs pixel selection through XY-address-based switching operations by using the vertical scanning circuit 303 and the horizontal scanning circuit 304. A light voltage signal and noise voltage signal for each pixel amplified by transistors are output to analog output terminals S and N via the column signal lines 307 and 306 and the analog output signal lines 308 and 309.

When the chip select signal input terminal CS is turned ON, the optical voltage signal and the noise voltage signal of an imaging device according to internal scanning are output from the analog output terminals S and N, respectively. An S signal output selector analog switch (transfer switch), an N signal output selector analog switch (transfer switch), the column signal lines 306 and 307, and switching transistors for selecting the column signal lines 306 and 307 form a read scanning transmission circuit.

The vertical scanning circuit clock terminal CLKV inputs a clock of the vertical scanning circuit 303, and the vertical scanning circuit start signal terminal VST inputs a start signal of the vertical scanning circuit 303. When the imaging control unit 112 sets to HIGH a signal input to the vertical scanning circuit start signal terminal VST and then inputs a clock from the vertical scanning circuit clock terminal CLKV, the row selection signals are sequentially enabled in order of V1, V2, ..., and Vm. When vertical scanning is activated, the imaging control unit 112 sets to LOW the signal input to the vertical scanning circuit start signal terminal VST.

The horizontal scanning circuit clock terminal CLKH inputs the clock of the horizontal scanning circuit 304, and the horizontal scanning circuit start signal terminal HST inputs the start signal of the horizontal scanning circuit 304. When the imaging control unit 112 sets to HIGH a signal input to the horizontal scanning start signal terminal HST and then sets to HIGH a signal input to the horizontal scanning circuit clock terminal CLKH, the row selection signal is sequentially enabled in order of H1, H2, ..., and Hn. When horizontal scanning is activated, the imaging control unit 112 sets to LOW the signal input to the horizontal scanning start signal terminal HST.

When a row selection signal V1 of the vertical scanning circuit 303 is enabled, (n, 1) is selected from the pixel group (1, 1) of one horizontal line connected with the row selection signal V1, and the voltage signals S and N are output to the column signal lines 306 and 307. By enabling the row selection signal of the horizontal scanning circuit 304 in order of H1, H2, ..., and Hn, the signal output switch and noise output switch provided for each column signal line are sequentially turned ON. Thus, voltage signals S and N of pixels 302 of one horizontal row are output to the analog output terminals S and N via the analog output signal lines 308 and 309, respectively. By performing similar horizontal scanning up to a row selection signal Vm, pixel outputs of all pixels can be obtained.

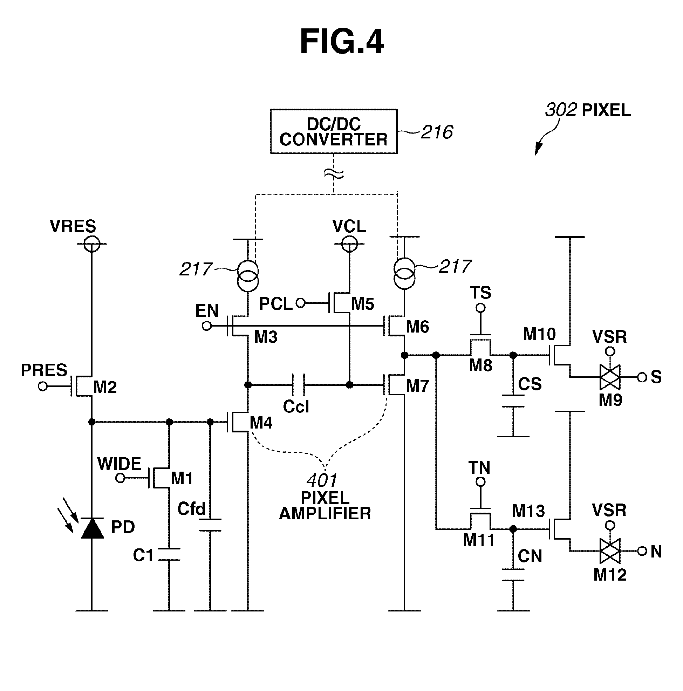

A configuration of each of the above-described pixels 302 will be described below with reference to Fig. 4. The pixel 302 includes transistors M 1 to M 13.

A photo diode PD converts light into an electrical signal. A capacitance Cfd is a floating diffusion capacitance for accumulating charge carriers. A switch M2 is a reset MOS transistor (reset circuit) for discharging charge carriers accumulated in the capacitance Cfd. A switch M2 is controlled by a PRES signal. A photo diode PD and the capacitance Cfd are reset by a voltage VRES which is applied thereto. A switch M 1 is used to connect or disconnect between an additional capacitance C1 for accumulating charge carriers and the photo diode PD. The switch M1 is controlled by a WIDE signal of the imaging control unit 112

A pixel amplifier M4 in the first stage of a clamp circuit enables obtaining an electrical signal according to the amount of charge carriers accumulated in the capacitance Cfd. The clamp circuit includes a clamp capacitance Ccl and a switch M5 for applying a clamp voltage VCL to the clamp capacitance Ccl. A switch M5 is turned ON and OFF by a PCL signal from the imaging control unit 112. The clamp circuit in which the clamp voltage VCL is applied to the clamp capacitance Ccl removes kTC noise from the electrical signal. Then, the pixel amplifier M7 in the last stage amplifies the signal.

The electrical signal acquired via the pixel amplifiers 401 is held by the sample-hold circuit. The sample-hold circuit is a holding unit connected with the pixel amplifiers 401, and configured to hold the electrical signal acquired via the pixel amplifiers 401. The pixel 302 includes two sample-hold circuits: one is for the light signal and the other is for fixed pattern noise (FPN). A capacitance CS is for the light signal and a capacitance CN is for FPN.

Switches M8 and M 11 are controlled by signals TS and TN from the imaging control unit 112 to determine timing of holding the electrical signal in the capacitances CS and CN, respectively. Source follower amplifiers M 10 and M 13 amplify the light signal and FPN, respectively. The electrical signal acquired through light reception is sample-held by a photoelectric charge sample-hold circuit, and the signal voltage corresponding to FPN is sample-held by a noise sample-hold circuit.

The two sample-held electrical signals are applied to the column signal line 307 for photoelectric charge and the column signal line 306 for noise according to control of the switches M9 and M 12, respectively, by a signal VSR from the vertical scanning circuit 303. Further, the electrical signals are output from the column signal lines 307 and 306 to the outside via the analog output lines 308 and 309, respectively. By obtaining a difference between the output signals by using the differential amplifier 202, FPN due to circuit factor in the image sensor 111 can be removed.

Each of the pixel amplifiers 401 is arranged in the preceding and subsequent stages of the clamp circuit. The pixel amplifier M4 in the first stage connected with the photo diode PD enables acquiring an electrical signal according to the amount of charge carriers accumulated in the capacitance Cfd. The pixel amplifier M4 in the first stage is connected with the switch M3 for connecting or disconnecting between a constant current source 217 and the pixel amplifier M4.

The ON/OFF timing of the switch M3 is controlled by an EN signal from the imaging control unit 112. The switch M3 connects or disconnects between the constant current source 217 and the pixel amplifier M4. When the signal output unit 211 of the imaging control unit 112 sets the EN signal to HIGH, the switch M3 turns ON to connect between the constant current source 217 and the pixel amplifier M4. Then, from the constant current source 217, a current flows to the pixel amplifier M4 to activate the pixel amplifier M4. The activated pixel amplifier M4 enables acquiring an electrical signal according to the amount of charge carriers.

Likewise, the ON/OFF timing of the switch M6 is controlled by the EN signal. The switch M6 connects or disconnects between a constant current source 217 and the pixel amplifier M7 in the subsequent stage. When the imaging control unit 112 sets the EN signal to HIGH, the pixel amplifiers M4 and M7 are simultaneously activated.

The EN signal is output from the signal output unit 211 of the imaging control unit 112 to each pixel. The pixel amplifiers 401 are activated at timing when charge carriers produced in the photo diode PD are transferred to the sample-hold circuit. The activation timing of the pixel amplifiers 401 (hereinafter referred to as pixel amplifier activation timing) is not the same for all pixels, i.e., it is different for each sub area 301 illustrated in Fig. 3. The pixel amplifiers 401 are activated in order of the four rectangular semiconductor substrates 201A to 201D (or A/D conversion area) . The pixel amplifiers 401 are activated at the same timing for all pixels in the sub area 301.

If the pixel amplifiers 401 for all pixels of the image sensor 111 are simultaneously activated, a large rush current occurs. This may cause electromagnetic wave generation or image quality deterioration by increased radiation noise. By shifting the pixel amplifier activation timing for each sub area 301, a rush current can be restrained.

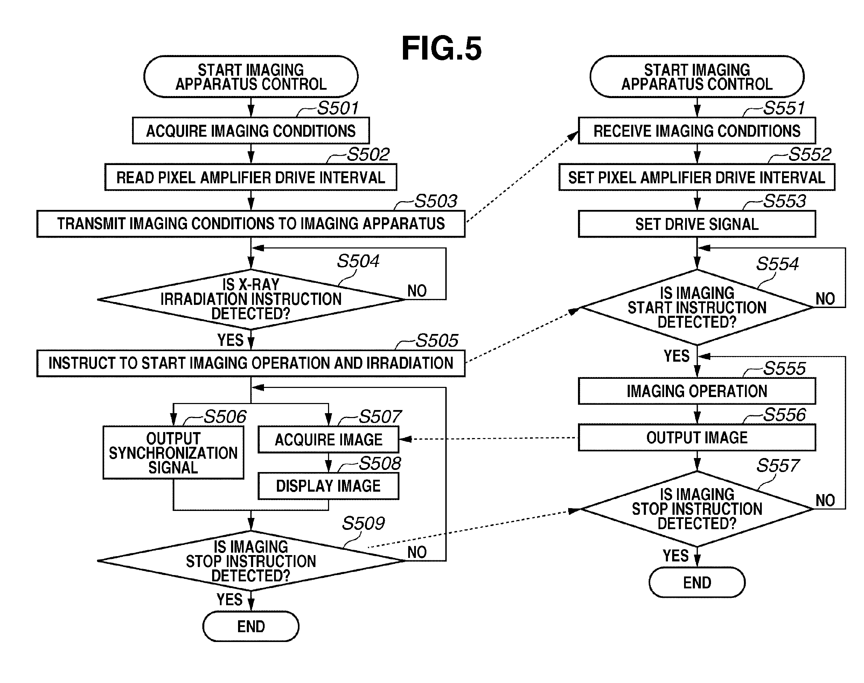

A processing flow by the X-ray imaging system having the above-described configuration will be described below with reference to the flowchart illustrated in Fig. 5. A processing flow of the control apparatus 100 is illustrated in the flowchart on the left-hand side of Fig. 5, and a processing flow of the imaging apparatus 101 is illustrated in the flowchart on the right-hand side of Fig. 5. The dotted arrows drawn between the two flowcharts in Fig. 5 indicate the order of processing between the control apparatus 100 and the imaging apparatus 101. Specifically, the processing on the arrowhead is performed in response to the processing on the starting point of each arrow.

In step S501, the imaging condition setting unit 108 of the control apparatus 100 acquires imaging conditions input to the operation unit 105. Example settings for the imaging apparatus 101 include the frame rate and gain settings, the presence or absence of binning drive, a setting of pulsed or continuous X-ray imaging. Example settings for the X-ray generator 102 include the tube voltage, tube current, and irradiation time settings, and a setting of pulsed or continuous irradiation. Settings for the image processing unit 109 include parameter settings for image processing, and settings for the display control unit 110 include parameter settings for display processing.

In step S502, the amplifier setting unit 107 acquires setting information regarding the pixel amplifier activation timing stored in a storage unit (not illustrated). The setting information regarding the activation timing includes information about the activation order and activation interval for each sub area 301. If either one of the activation order and activation interval settings is predetermined, only the other setting may be transmitted. For example, if the activation order setting is predetermined, only the activation interval setting may be transmitted. The setting information may be changed or updated in response to an input to the operation unit 105.

In step S503, the imaging condition setting unit 108 instructs the imaging apparatus 101, the X-ray generator 102, and the image processing unit 109 and the display control unit 110 of the control apparatus 100 to set imaging conditions. The amplifier setting unit 107 instructs the imaging apparatus 101 to set the pixel amplifier activation timing. The relevant instructions are transmitted from a communication device (not illustrated) to the imaging apparatus 101 via the command control interface 212. Upon reception of the relevant instructions, the imaging apparatus 101 performs processing in step S551.

In step S504, the control apparatus 100 determines whether the X-ray irradiation switch (not illustrated) is pressed. When the depression of the X-ray irradiation switch is not detected (NO in step S504), the control apparatus 100 repeats the determination process and waits until the X-ray irradiation switch is pressed. When the depression of the X-ray irradiation switch is detected (YES in step S504), the control apparatus 100 determines that X-ray irradiation is instructed by the user.

When it is determined that X-ray irradiation is instructed by the user (YES in step S504), the processing proceeds to step S505. In step S505, the control apparatus 100 instructs the imaging apparatus 101 to start the imaging operation via the synchronization control unit 106. Upon reception of the exposure permission signal 215 from the imaging apparatus 101, the control apparatus 100 instructs the X-ray generator 102 to generate X-ray. When the synchronization control unit 106 transmits the external synchronization signal 214 to the imaging apparatus 101 and the X-ray generator 102, the imaging apparatus 101 performs imaging operation and the X-ray generator 102 performs X-ray irradiation in a synchronized manner, thus achieving X-ray imaging. In this way, the imaging apparatus 101 performs determination processing in step S554.

In step S506, the synchronization control unit 106 outputs a synchronization signal. In step S507, the image processing unit 109 acquires output of the captured image from the imaging apparatus 101 and then performs predetermined image processing. In step S508, the display control unit 110 displays on the display unit 104 the captured image that has undergone the image processing.

The processing repeats the above-described steps S506, S507, and S508 until an imaging stop instruction is detected. The image processing unit 109 performs the image acquisition processing in step S507 according to image output processing in step S556 by the imaging apparatus 101.

In step S509, the synchronization control unit 106 determines whether the imaging stop instruction is detected via the operation unit 105. When the imaging stop instruction is detected (YES in step S509), imaging is stopped. Specifically, the synchronization control unit 106 instructs the imaging apparatus 101 and the X-ray generator 102 to stop imaging. Upon reception of the relevant instruction, the imaging apparatus 101 performs processing in step S557.

Processing performed by the imaging apparatus 101 will be described below.

In step S551, the imaging control unit 112 receives the setting information regarding the imaging conditions from the control apparatus 100. The imaging control unit 112 performs this processing in response to the processing for transmitting the setting information in step S503 by the control apparatus 100. The setting information includes the setting of the pixel amplifier activation timing acquired by the amplifier setting unit 107.

In step S552, the signal setting unit 209 sets the timing of outputting the signal to be output to the image sensor 111, based on the setting information regarding the pixel amplifier activation timing. For example, as illustrated in Fig. 2, the signal setting unit 209 sets the timing of outputting the EN signals to the rectangular semiconductor substrates 201A, 201B, 201C, and 201D of the imaging area 219 in a shifted manner at 20-microsecond (µs) intervals in this order.

In step S553, the signal setting unit 209 sets the driving signal based on the received setting information regarding imaging conditions. Specifically, according to the frame rate and gain setting and the presence or absence of binning drive, the signal setting unit 209 performs processing for selecting the HIGH/LOW state of a signal WIDE and the HIGH/LOW state of a binning instruction signal.

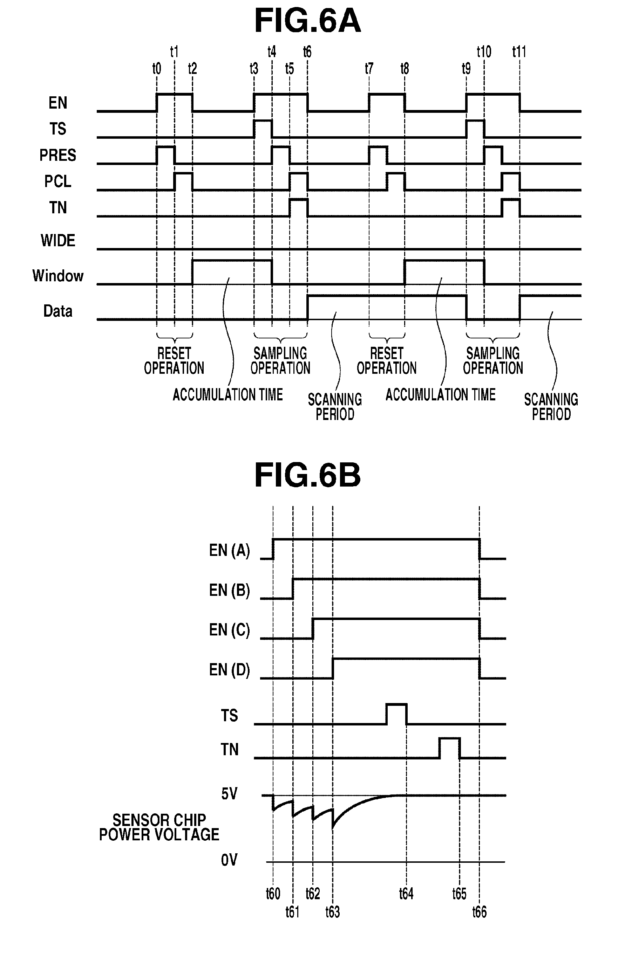

In step S554, the imaging apparatus 101 determines whether an imaging start instruction from the control apparatus 100 is detected. When the imaging start instruction is not detected (NO in step S554), the imaging apparatus 101 repeats the determination process and waits until the instruction is detected. When the imaging start instruction is detected (YES in step S554), the processing proceeds to step S555. In step S555, the imaging apparatus 101 starts the imaging operation. The imaging operation will be described below with reference to Figs. 6A and 6B.

In step S556, a signal acquired by the imaging operation is output from the image sensor 111, amplified by the differential amplifier 202, and converted into digital data by the A/D converter 203. The image generation unit 204 generates captured image data based on the digital data. Then, the output control unit 207 outputs the captured image data to the control apparatus 100 via the image data interface 208. Upon reception of the captured image data, the control apparatus 100 performs image acquisition processing in step S507.

In step S557, the signal control unit 210 determines whether an imaging stop instruction from the control apparatus 100 is detected. When the imaging stop instruction is not detected (NO in step S557), the signal control unit 210 performs control to repeat the imaging operation in step S555 and the image output processing in step S556. When the imaging stop instruction is detected (YES in step S557), the signal control unit 210 outputs an imaging stop signal to the signal output unit 211 and outputs an image generation and output processing stop signal to the generation unit 204.

The imaging operation performed by the signal control unit 210 and the signal output unit 211 of the imaging control unit 112 in step S555 (see Fig. 5) will be described below with reference to the pixel circuits illustrated in Fig. 4 and the timing chart illustrated in Fig. 6A. Figs. 6A and 6B are timing charts illustrating drive timings at the time of moving image capturing while restricting X-ray window with a fixed frame rate. Control signal timings in capturing a moving image until charge carriers are sample-held in the light signal hold capacitance CS and the noise signal hold capacitance CN will be described below with reference to Fig. 6A.

A reset operation started at timing t0 will be described below. The reset operation includes resetting and clamping. At the timing t0, the signal output unit 211 sets the EN signal to HIGH to activate the pixel amplifiers M4 and M7. Then, the signal output unit 211 sets the PRES signal to HIGH to connect the photo diode PD to a reference voltage to reset it. When the signal output unit 211 sets the PRES signal to LOW to end a reset operation, a reset voltage is applied to the pixel amplifier M4 side of the clamp capacitance Ccl.

At timing t1, the signal output unit 211 sets the PCL signal to HIGH to turn ON the clamp switch M5. A reference voltage VCL is applied to the pixel amplifier M7 side of the clamp capacitance Ccl. The signal output unit 211 turns OFF the clamp switch M5, charge carriers corresponding to the voltage for a difference between the reference voltage VCL and the reference voltage VRES are accumulated in the clamp capacitance Ccl, and clamping is completed.

When the reset operation ends, at timing t2, charge accumulation is started in a photoelectric conversion unit including the photo diode PD and the floating diffusion capacitance Cfd.

In the tiled CMOS imaging devices, to prevent image deviation due to switching timing differences between imaging devices and between scanning lines at the time of capturing the moving image, the reset operation is collectively applied to all pixels of each tiled imaging device at the same timing and in the same time period. Then, by collective charge accumulation, photoelectric charge carriers (generated in the photo diode PD) can be accumulated in the floating diffusion capacitance Cfd.

During the reset operation from the timings t0 to t2, although reset noise (kTC noise) is generated in the photoelectric conversion unit, by applying the reference voltage VCL to the pixel amplifier M7 side of the clamp capacitance Ccl of the clamp circuit, reset noise can be removed.

A sampling operation started at timing t3 will be described below. The signal output unit 211 sets the EN signal to HIGH to turn ON a selection switch 1 (M3) and a selection switch 2 (M6). The charge carriers accumulated in the floating diffusion capacitance Cfd are converted into a voltage and output as a voltage to the clamp capacitance Ccl by the pixel amplifier M4 operating as a source follower amplifier. Although the output of the pixel amplifier M4 contains reset noise, the voltage is output to the pixel amplifier M7 as a light signal with reset noise removed since the reference voltage VCL is applied to the pixel amplifier M7 side of the clamp circuit during the reset operation.

Then, the signal output unit 211 sets a sample-hold TS control signal to HIGH to turn ON a sample-hold switch S (M8). As a result, the light signal is collectively transmitted to the light signal hold capacitance CS via the pixel amplifier M7. At timing t4, the signal output unit 211 sets the TS signal to LOW to turn OFF the sample-hold switch S (M8). The photoelectric charge signal is sample-held by the light signal hold capacitance CS. Then, the signal output unit 211 sets the reset PRES signal to HIGH to turn ON the reset switch (M2). The floating diffusion capacitance Cfd is reset to the reference voltage VRES. At timing t5, the signal output unit 211 sets the reset PRES signal to LOW to complete resetting.

Then, the signal output unit 211 sets the PCL signal to HIGH. Charges of the voltage difference between the voltage VCL and the voltage VRES having reset noise superimposed thereon are accumulated in the clamp capacitance Ccl. The signal output unit 211 further sets the TN signal to HIGH simultaneously with the PCL signal to turn ON a sample-hold switch N (M 11), so that the noise signal when the reference voltage VCL is applied to the pixel amplifier M7 side of the clamp circuit is transmitted to the noise signal hold capacitance CN.

Subsequently, at timing t6, the signal output unit 211 sets the TN signal to LOW to turn OFF the sample-hold switch N (M 11), so that the noise signal is sample-held in the noise signal hold capacitance CN. Then, the signal output unit 211 sets the EN and PCL signals to LOW to complete the sampling operation. The sampling operation is performed collectively for all pixel areas.

After the sampling operation, at timing t7, the signal output unit 211 performs the reset operation again to start charge accumulation in the photo diode PD for the following frame.

Light signal and noise signal scanning is performed for each pixel. When the signal output unit 211 turns ON a transfer switch S (M9) and the transfer switch N (M12), voltages of the light signal hold capacitance CS and the noise signal hold capacitance CN are transmitted to the light signal output line and the noise signal output line via the pixel amplifiers M 10 and M 13, respectively.

The signals transmitted to the noise signal output line and the light signal output line are subtracted by respective differential amplifiers 202 connected to the noise signal output line and the light signal output line. Thus, FPN caused by thermal noise, 1/f noise, temperature difference, and process variation in the pixel amplifiers 401 is removed. The signals can be transmitted between the time when sample holding is ended (t6) and the time when the photoelectric charge signal and the noise charge signal in the following frame are sample-held in the light signal hold capacitance CS and the noise signal hold capacitance CN, respectively (t9).

In the pixel circuits illustrated in Fig. 4, charge accumulation in the photo diode PD is started when the signal output unit 211 sets the PCL signal to LOW to complete clamping (t2 and t6 in Fig. 6A). Charge carrier accumulation is ended when the signal output unit 211 sets the TS signal to LOW to sample-hold the light signal (t4).

As described above, by inserting a reset operation or sampling operation for starting a charge accumulation time between the sampling operation for sample-holding the light signal and the sampling operation for sample-holding the noise signal, the charge accumulation time can be limited. Referring to Fig. 6A, by inserting a reset operation (t7), the charge accumulation time is limited to a period between timings t8 and t10 which equals a period between the timings t2 and t4 while the light signal sample-holding interval ranges from the timing t4 to the timing t10.

When operations of the pixel circuits illustrated in Fig. 6A are simultaneously performed in all pixel areas, the EN signals are set to HIGH (t0, t3, t7, and t9) and then the pixel amplifiers 401 (M4 and M7) for all pixels (see Fig. 4) are simultaneously activated. Thus, a rush current occurs. If a large rush current flows in a signal line, unnecessary radiation occurs from the power supply line or signal lines within the imaging apparatus 101. Further, a rush current may cause a rapid voltage drop in the power supply voltage of a sensor chip (substrate), possibly causing an operation stop or malfunction. In addition, since it takes time until the dropped power supply voltage of the sensor chip stabilizes again, the power supply to the sensor chip becomes unstable. Accordingly, by shifting the pixel amplifier activation timing for each sub area 301, a rush current can be restrained.

Operations of the pixel amplifiers 401 and sample-hold operations during the sampling operation at the time of capturing the moving image will be described below with reference to Fig. 6B. Fig. 6B is a timing chart illustrating driving waveforms, and schematically illustrates a waveform of the power supply voltage of the sensor chip of the imaging apparatus 101. Signals EN(A) to EN(D) for driving the pixel amplifiers 401 are illustrated for each of the rectangular semiconductor substrates 201A to 201D, respectively. For example, the EN(A) signal corresponds to the rectangular semiconductor substrate 201A illustrated in Fig. 2. The bottom graph in Fig. 6B schematically illustrates variation in the power supply voltage of the sensor chip in synchronization with the timing chart.

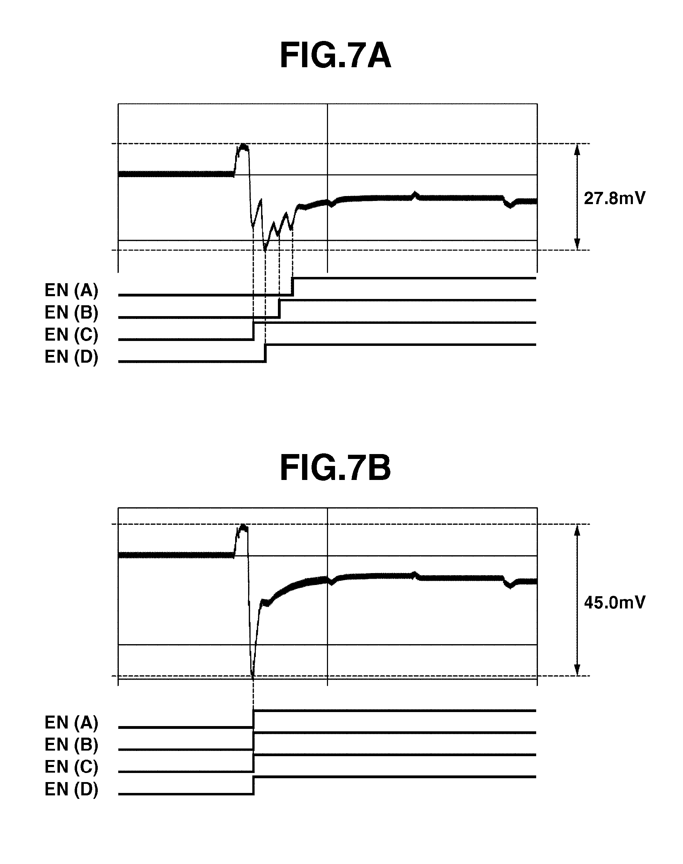

The imaging control unit 112 transmits to the image sensor 111 driving signals including the EN (A) to EN (D) signals (see Fig.7), the TS signal, and the TN signal. The imaging control unit 112 changes the timing of asserting the EN signals for each sub area 301.

As illustrated in Fig. 6B, the signal control unit 210 performs control so that the EN signals for each pixel area (sub area) 301 are turned ON at different timings. The signal output unit 211 turns ON the EN (A) signal for a sub area 301A (t60). After a predetermined time period has elapsed, the signal output unit 211 turns ON the EN(B) signal for a sub area 301B (t61). After a predetermined time period has elapsed, the signal output unit 211 turns ON the EN (C) signal for a sub area 301C (t62). After a predetermined time period has elapsed, the signal output unit 211 turns ON the EN (D) signal for a sub area 301D (t63). After the pixel amplifiers 401 in respective sub areas 301 are activated and a predetermined time has elapsed, the signal output unit 211 sets an electrical signal acquired via the pixel amplifiers 401 to the sample-hold circuit (holding unit) to restrain it (t64). Then, the signal output unit 211 sample-holds the FPN signal (t65). After a series of sampling operations is completed, the signal output unit 211 collectively turns OFF the EN(A) to EN(D) signals for all of the sub areas 301A to 301D (t66).

The signal output unit 211 collectively performs control on all of the pixel areas except for the activation timing of the EN (A) to EN (D) signals (see Fig.6B). This enables preventing image deviation due to switching timing differences between substrates and between scanning lines at the time of capturing the moving image. Further, the signal output unit 211 controls the timing of ending of light signal charge transfer to the sample-hold circuit (t44) and the timing of ending of noise signal charge transfer thereto (t45) so that the power supply voltage of the image sensor 111 remains stable.

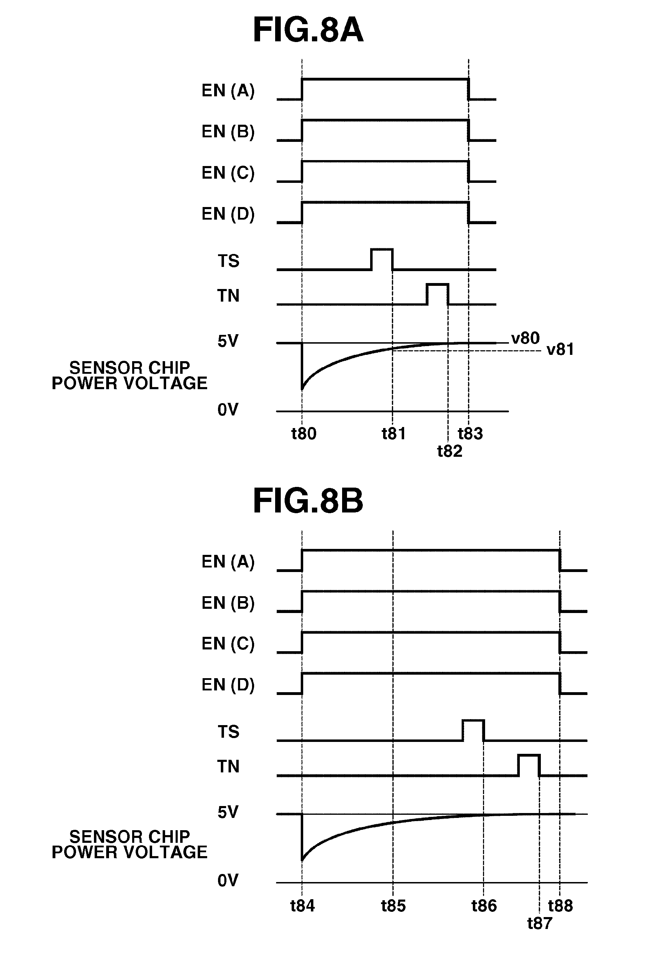

Also in the above-described processing, the power supply voltage of the image sensor 111 slightly drops when the EN signals are turned ON. However, if the imaging control unit 112 shifts the timing of turning ON the EN signals for respective sub areas 301, a rush current can be reduced. Therefore, in comparison with the control method (see Figs. 7B, 8A, and 8B) for collectively turning ON the EN signals for all of the sub areas 301A to 301D, the above-described control enables restraining of voltage drop in the image sensor 111.

Effects of processing according to the present exemplary embodiment will be described below with reference to Figs. 7A and 7B. Figs. 7A and 7B illustrate power supply voltage waveforms of the sensor chip when the image sensor 111 having the pixels illustrated in Fig. 4 is actually used, and states of the EN signals at the same time. Referring to Figs. 7A and 7B, the horizontal axis represents time and the vertical axis represents the power supply voltage of the sensor chip. Apower supply voltage waveform for each sensor chip is measured at a measuring point 218 where the power is supplied to the pixel area (sub area) 301C of the rectangular semiconductor substrate 201 illustrated in Fig. 3.

Referring to Figs. 7A and 7B, the difference between the power supply voltage before EN signals are set to HIGH and the power supply voltage after they are set to HIGH (steady state) is caused by an internal resistance of the circuit. An ideal waveform is as illustrated in Fig. 6B.

After the four EN signals are turned ON, a faint voltage variation is seen in the middle of the graph because the TS signal is turned ON to sample-hold the light signal. At subsequent timing, the voltage slightly swings upward because the PRES signal is turned ON to perform resetting. Then, the voltage slightly drops at certain timing because the PCL signal is turned ON to apply the clamp voltage to the clamp capacitance Ccl and then the TN signal is turned ON to sample-hold the FPN signal. Thus, the effect of voltage drop by the pixel amplifiers 401 is larger than the effect of other elements.

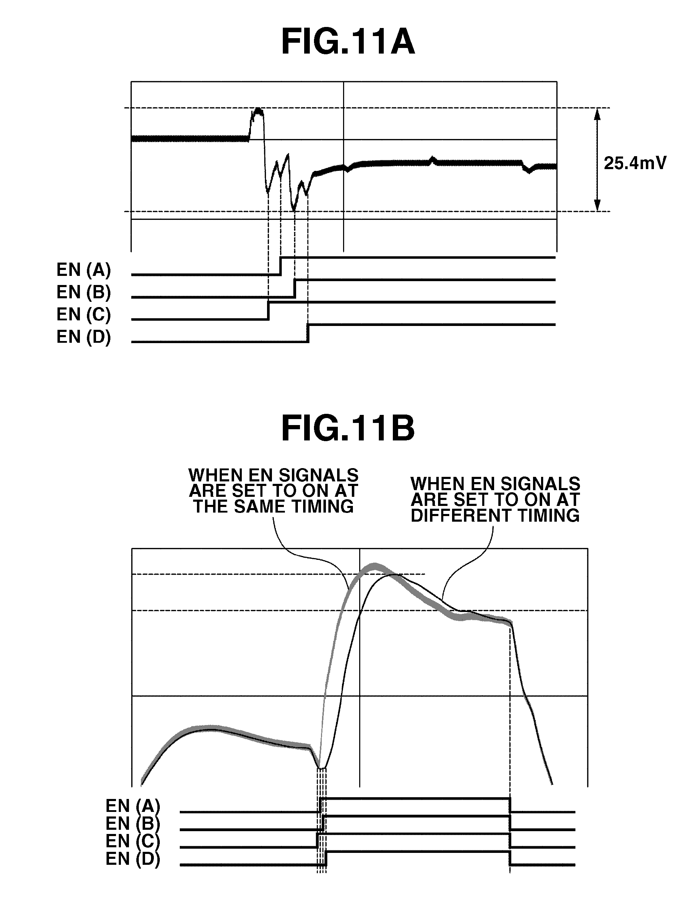

Fig. 7A illustrates a case where control according to the present exemplary embodiment is carried out. More specifically, it represents a waveform of the power supply voltage when the imaging control unit 112 shifts the timing of turning ON the EN signals to the pixel areas 301C, 301D, 301B, and 301A (see Fig. 11A) at 30-µs intervals in this order.

Fig. 7B illustrates a comparative example, i.e., a power supply voltage waveform of the sensor chip when the imaging control unit 112 collectively turns ON the EN signals for all of the pixel areas 301.

As illustrated in Figs. 7A and 7B, by shifting the timing of turning ON the EN signals, power supply voltage drop in the sensor chip can be restrained. The measurement result according to the present exemplary embodiment (see Fig. 7A) indicates that a difference between the peak and bottom voltages is 27.8 mV while the measurement result according to the comparative example (see Fig. 7B) indicates that the difference is 45.0 mV. Thus, voltage drop is restrained by the control according to the present exemplary embodiment.

Other comparative examples for describing the effect of the present exemplary embodiment will be described below with reference to Figs. 8A and 8B. The graph in Fig. 8A shows a case where the EN signals are collectively turned ON for all of the sub areas 301 as illustrated in Fig. 7B, and sample-holding is performed with an unstable power supply voltage of the sensor chip in a drive sampling operation portion.

In this case, there arises a difference between a power supply voltage v81 of the sensor chip at the time of light signal charge transfer to the sample-hold circuit (t81) and a power supply voltage v80 of the sensor chip at the time of FPN signal charge transfer thereto (t82). Thus, the difference in the power supply voltage of the sensor chip will be superimposed onto a captured image as an offset.

The power supply voltage drop in the sensor chip also becomes different depending on the amount of light. In the example pixel circuits illustrated in Fig. 4, the capacitors Cfd and C1 discharge when the photo diode PD receives light. Therefore, when all pixels of the pixel amplifiers M4 and M7 are simultaneously activated by setting the EN signal to HIGH, the more amount of light, the larger voltage drop in the sensor chip. Further, within the rectangular semiconductor substrates 201A to 201D, a rush current flow produces voltage drop because of the wiring resistance of the power supply line due to fine wiring. Therefore, depending on the amount of incident light, the power supply voltage at the time of sample-holding differs for each rectangular semiconductor substrate. This effect appears in a captured image as level differences between the tiled rectangular semiconductor substrates.

One possible solution for the above-described problem of the difference of the power supply voltages of the sensor chip between when sample-holding the light signal and when sample-holding the noise signal, is to wait for a sufficient time period for voltage stabilization after the pixel amplifiers 401 are activated and perform sample-holding. The graph in Fig. 8B shows a case where the EN signals are collectively turned ON for all sub areas 301 as illustrated in Fig. 7B, and drive sampling operation is performed with a stable power supply voltage supplied to the substrates.

In comparison with the graphs Figs. 6A and 8A, the graph in Fig. 8B indicates a long time period from the time when the EN signals are turned ON (t84) up to the time when light signal charge and noise signal charge transfers to the sample-hold circuit are completed (t86 and t87). Therefore, the Fig. 8B case is useful in that there arises no difference between the power supply voltage of the sensor chip at the end of light signal charge transfer to the sample-hold circuit (t86) and the power supply voltage of the sensor chip at the end of noise signal charge transfer thereto (t87).

In this case, however, since the time period during which the EN signals are turned ON over the entire image sensor 111 becomes longer, the power consumption increases in comparison with the operations according to the present exemplary embodiment (see Fig. 6A). Further, since a processing time between charge accumulation and sample-holding is prolonged, it becomes difficult to increase the frame rate. Further, since the EN signals are simultaneously turned ON for the entire image sensor 111, the peak level of a rush current cannot be restrained.

As above-described, in the first exemplary embodiment, the signal output unit 211 performs control to sequentially turn ON the power supply of the pixel amplifiers 401 of the imaging apparatus 101 at different timings for respective sub areas 301. This enables restraining of rush current to reduce unnecessary radiation and noise resulting from the amount of rush current. Further, since a rapid voltage drop by rush current is also restrained, the instability of the power supply to the sensor chip is remedied.

The imaging control unit 112 determines at least one of the activation order and activation interval of the above-described pixel amplifiers 401 based on an instruction of an external control apparatus, thus achieving drive operations which restrains rush current, depending on a situation.

The above-described control further enables restraining of offset noise due to the difference between the power supply voltage at light signal charge transfer and the power supply voltage at FPN signal charge transfer while reducing the processing time between charge accumulation and sample-holding.

When a plurality of semiconductor substrates is bonded together, level differences may appear in an image due to differences in the amount of light received by the pixels of each substrate. However, by restraining voltage drop, level differences in the image can be restrained.

Further, in comparison with a case where the pixel amplifiers 401 for all imaging areas are collectively activated, a rush current can be restrained, thus reducing the power consumption.

Furthermore, with an X-ray image sensor 111 having a comparatively large imaging areas like the one in the present exemplary embodiment, recovery from voltage drop takes time because of a large wiring size. Applying the control and change of the above-described pixel amplifier activation timing according to the present exemplary embodiment to such an X-ray image sensor 111 is remarkably effective in restraining noise superimposed on an image and improving the frame rate.

In a second exemplary embodiment, the pixel amplifier activation order for each sub area is based on the descending order of the wiring resistance from the power supply to each sub area. This enables activating of the pixel amplifiers 401 sequentially from a sub area having a large wiring resistance and showing hard voltage recovery, which restrains voltage drop to further extent than the first exemplary embodiment.

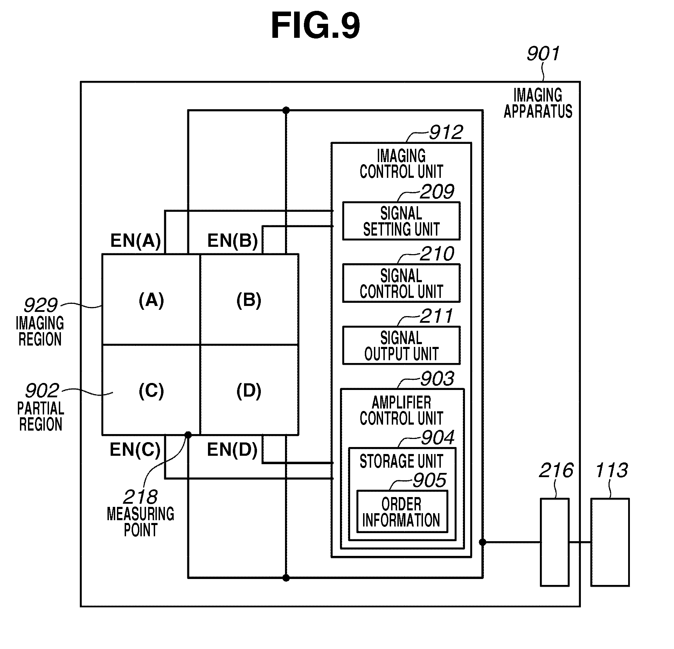

A configuration and processing of an X-ray imaging system according to the present exemplary embodiment will be described below, focusing on differences from the first exemplary embodiment, with reference to Figs. 9 to 11.

A configuration of an imaging apparatus 901 will be described below with reference to Fig. 9. Fig. 9 illustrates differences from the first exemplary embodiment. Similar to the first exemplary embodiment, the image sensor 111 is formed of a plurality of rectangular semiconductor substrates bonded together. An imaging area 929 of the image sensor 111 is divided into four sub areas 902A, 902B, 902C, and 902D.

An imaging control unit 912 of the imaging apparatus 901 includes an amplifier control unit 903 for controlling the pixel amplifier activation order. The signal control unit 210 performs control to output the EN signals (EN(A), EN(B), EN(C), and EN (D) signals) separately for respective sub areas 902. The signal output unit 211 outputs the EN(A), EN(B), EN(C), and EN(D) signals to the sub areas 902A, 902B, 902C, and 902D, respectively.

The amplifier control unit 903 includes a storage unit 904 for storing order information 905 indicating the pixel amplifier activation order for the sub areas 902A, 902B, 902C, and 902D.

The order information 905 is generated in advance according to the wiring resistance of the wire for supplying the power to each pixel from the DC/DC converter (power supply unit) 216. The resistance value may be directly measured or set according to the wiring length. When only one power supply unit is provided, the sub area 902 at the most distant position from the power supply is regularly processed earlier than any other sub areas 902. The order information 905 includes predetermined values of the pixel amplifier activation interval for each sub area 902.

When a plurality of semiconductor substrates is included in one sub area 902 and the power is independently supplied from the DC/DC converter 216 to each semiconductor substrate, a plurality of wiring is provided in each sub area 902. In this case, it is preferable to calculate an average wiring value of the wire connected to each substrate and determine the activation order for each sub area 902 based on the average wiring value.

The amplifier control unit 903 acquires the order information 905 from the storage unit 904, and sets the output order of the EN signals to the image sensor 111 based on the order information 905. The order information 905 includes predetermined values and can be changed based on a user instruction. In that case, similar to the first exemplary embodiment, the amplifier control unit 903 sets the output order of the EN signals based on the setting information received from the control apparatus 100. The signal control unit 210 performs control to output earlier a driving signal to the pixel amplifiers 401 for pixels belonging to a sub area 902 having longer wiring connected to the pixel amplifiers 401, out of the plurality of sub areas 902 of the imaging area 929.

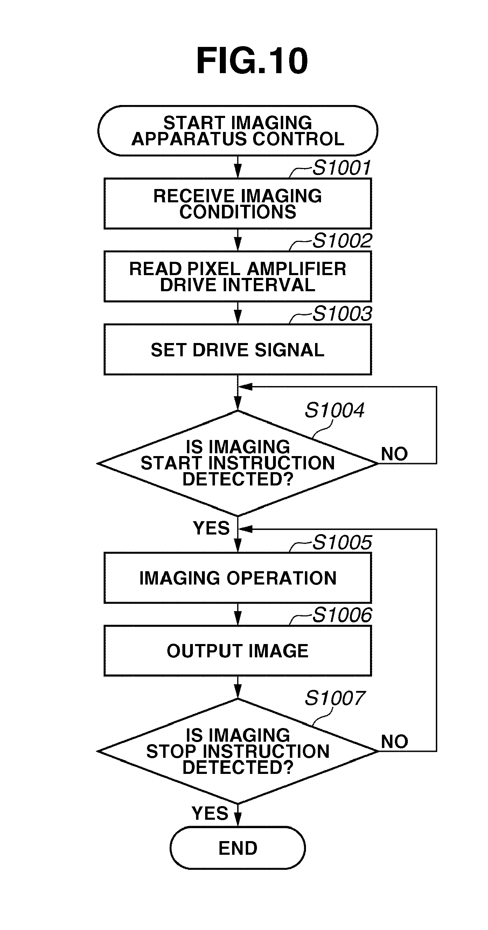

A control flow of the imaging apparatus 901 having the above-described configuration will be described below with reference to the flowchart in Fig. 10. As to processing similar to the first exemplary embodiment, descriptions will be omitted.

In step S1002, the amplifier control unit 903 reads the order information 905 from the storage unit 904. When setting information regarding the pixel amplifiers 401 is sent from the control apparatus 100, the amplifier control unit 903 also reads the setting information. When setting information regarding the pixel amplifiers 401 from the control apparatus 100 is not present, the amplifier control unit 903 sets the pixel amplifier activation interval and activation order based on the order information 905 in the storage unit 904. The setting information is stored in a memory, and used for driving signal setting by the signal setting unit 209.

In step S1003, the signal setting unit 209 sets as the timing of outputting the EN signals the imaging conditions received in step S1001 and amplifier setting information set by the amplifier control unit 903. The signal setting unit 209 performs control to output earlier a driving signal to the pixel amplifiers 401 for pixels belonging to a sub area 902 having longer wiring connected to the pixel amplifiers 401, out of the plurality of sub areas 902 of the imaging area 929. Subsequent processing is similar to the first exemplary embodiment.

The effect of the present exemplary embodiment will be described below with reference to Figs. 11A and 11B. Fig. 11A illustrates a voltage waveform when the imaging control unit 912 controls the timing of turning ON of the EN signals so as to activate earlier the respective sub areas 902 (pixel areas) having a longer distance from the power supply. With the example in Fig. 11A, the imaging control unit 912 shifts the timing of turning ON of the EN signals to the sub areas 902C, 902A, 902D, and 902B at 30-µs intervals in this order. The measuring point 218 is an equivalent position to that in the first exemplary embodiment, at which the voltage to be input to the sub area 902C is measured. Therefore, when the EN(C) and EN(D) signals for the sub areas 902C and 902D, are turned ON, the effect of large voltage drop is measured.

The measurement result in Fig. 11A indicates that a difference between the peak and bottom voltages is 25.4 mV which is smaller than the measurement result, 27.8 mV, in the first exemplary embodiment (see Fig. 7A). Thus, by activating earlier the pixel amplifiers 401 of the sub areas 902 having a larger wiring resistance value of the power supply wiring, a rush current can be further reduced, thus further restraining voltage drop.

Fig. 11B indicates waveforms of currents flowing in a semiconductor substrate in the present exemplary embodiment, and the states of the EN signals at the same time. Referring to Fig. 11B, the horizontal axis represents time and the vertical axis represents the current value. Fig. 11B illustrates a current waveform when the imaging control unit 912 shifts the timing of turning ON of the EN signals so as to activate earlier the pixel areas having a longer distance from the power supply. With the example in Fig. 11B, the imaging control unit 912 shifts the timing of turning ON of the EN signals to the sub areas 902C, 902A, 902D, and 902B at 30-µs intervals in this order. As a comparative example, Fig. 11B also illustrates a current waveform when the imaging control unit 912 collectively turns ON the EN signals for all of the pixel areas.

As illustrated by the two current waveforms in Fig. 11B, when the imaging control unit 912 shifts the timing of turning ON of the EN signals in this way, the current value flowing in the entire imaging apparatus 101 decreases, resulting in a decreased power consumption.

In this way, the present exemplary embodiment controls the pixel amplifier activation timing for each sub area 902 according to the wiring resistance value. In the case of imaging with a high frame rate, a total time during which the EN signals are asserted is longer, therefore the pixel amplifier activation time is prolonged which increases the power consumption. When the processing according to the present exemplary embodiment is performed in the case of a high frame rate, shifting the timing of turning ON of the EN signals achieves a remarkable effect resulting from reduced asserting time of the EN signals, thus effectively reducing the power consumption.

In a third exemplary embodiment, an interval at which the pixel amplifiers 401 for each sub area are activated in capturing the moving image is suitably determined based on the pixel values of the preceding frame.

As described in the first exemplary embodiment, power supply voltage drop in the sensor chip may also change depending on the amount of incident light. In the example of the pixel 302 in Fig. 4, since capacitors having the capacitance Cfd and the additional capacitance C1 discharge when the photo diode PD receives light, a larger amount of light causes a larger voltage drop when the pixel circuits are turned ON. For example, when a subject moves during capturing the moving image, the amount of incident light to the image sensor 111 entirely or locally changes, and the magnitude of power supply voltage drop in a rectangular semiconductor substrate changes. Even in this case, the operation timing of the pixel circuits is controlled. Thus, sample-holding can be performed with stable power supply voltage for the substrate.

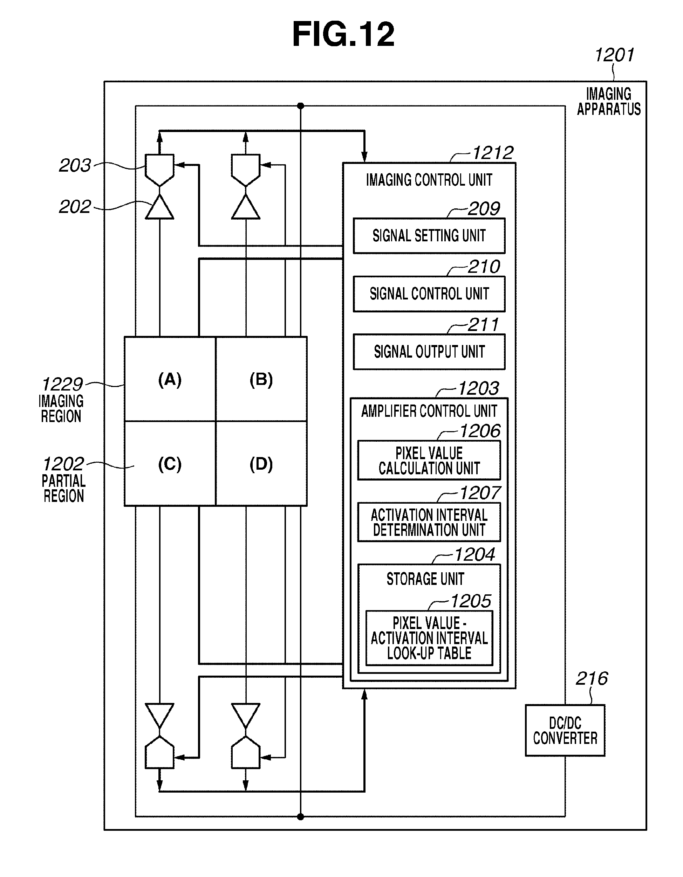

A configuration and processing of an X-ray imaging system according to the present exemplary embodiment will be described below, focusing on differences from the first and second exemplary embodiments, with reference to Figs. 12 to 14.

A configuration of an imaging apparatus 1201 will be described below with reference to Fig. 12. Fig. 12 illustrates the configuration centering on differences from the first and second exemplary embodiments. Unlike the first exemplary embodiment, the image sensor 111 according to the present exemplary embodiment is formed of one rectangular semiconductor substrate. An imaging area 1229 of the image sensor 111 is divided into four sub areas 1202A, 1202B, 1202C, and 1202D for each A/D converter 203.

An amplifier control unit 1203 of an imaging control unit 1212 includes a storage unit 1204, a pixel value calculation unit 1206, and an activation interval determination unit 1207. The imaging control unit 1212 has the functions of the image generation unit 204 (not illustrated in Fig. 12) according to the first exemplary embodiment.

The storage unit 1204 stores a pixel value and activation interval look-up table 1205. The look-up table 1205 arranges information regarding the pixel values and pixel amplifier activation interval for each sub area 1202 as table data. A pixel value is proportional to the amount of light received by each pixel of the image sensor 111. The larger the pixel values of a certain sub area 1202, the longer the interval until the pixel amplifiers 401 of the following sub area 1202 are activated because recovery from voltage drop takes longer time.

The pixel value calculation unit 1206 acquires a captured image generated by the image generation unit 204, and calculates a representative value of pixel values for each sub area 1202. The representative value may be a value that indicates the amount of light received by a pixel in a sub area 1202, such as an average, a median, and a sum total value. When all of the sub areas 1202 are the same in size, the average or median value is desirable. Although a maximum value can also be used, by using an average or median value, noise effect can be eliminated. For example, a maximum value excluding the top 5% may be used as a representative value.

The activation interval determination unit 1207 determines the pixel amplifier activation interval for each sub area 1202 referring to the calculated pixel values and the look-up table 1205.

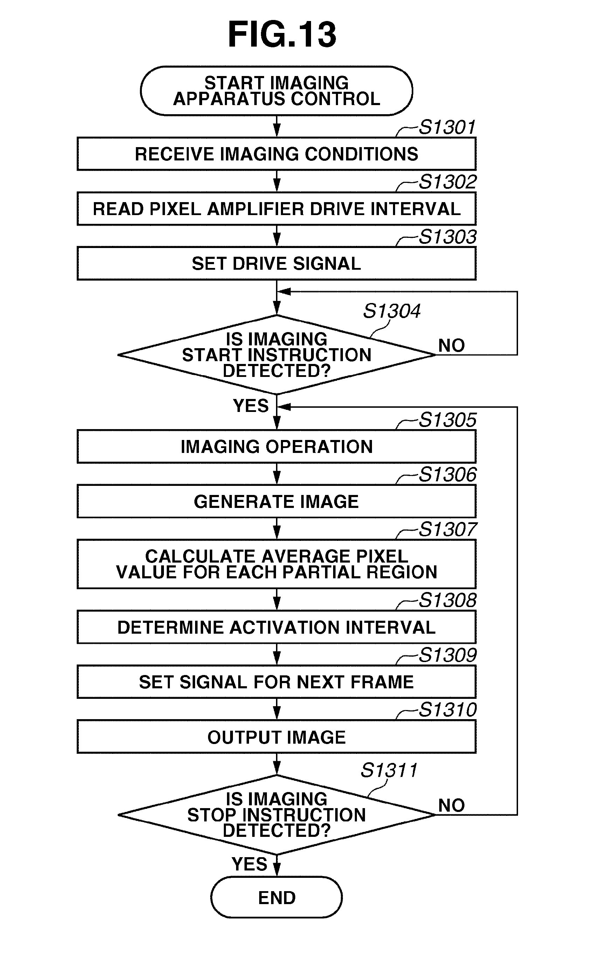

A processing flow of the imaging apparatus 1201 having the above-described configuration will be described below with reference to the flowchart illustrated in Fig. 13. As to processing similar to the first and second exemplary embodiments, descriptions will be omitted.

Processing in step S1301 is similar to the processing in step S1001 (see Fig. 10) in the second exemplary embodiment, and descriptions will be omitted.

In step S1302, the amplifier control unit 1203 sets initial values of the pixel amplifier activation order and activation interval based on the received setting information or the order information stored in the storage unit 1204.

Processing in steps S1303 to S1306 is similar to the processing in steps S1003 to S1006 (see Fig. 10) in the second exemplary embodiment, and descriptions will be omitted.

In step S1307, the pixel value calculation unit 1206 acquires captured image data and calculates an average of pixel values for each sub area 1202.

In step S1308, the activation interval determination unit 1207 determines the pixel amplifier activation interval for each sub area 1202 referring to the calculated pixel values and the look-up table 1205. The amplifier control unit 1203 transmits information about the determined activation interval to the signal setting unit 209.

In step S1309, the signal setting unit 209 sets the timing of asserting the EN signals according to the determined activation interval. When, in the present exemplary embodiment, the sub areas 1202A, 1202B, 1202C, and 1202D are activated in this order, the signal control unit 210 delays output by a determined time interval between the activation of the sub area 1202A and the activation of the sub area 1202B, and then instructs the signal output unit 211 to output the EN signals. The signal control unit 210 performs similar control for the time period between the activation of the sub area 1202B and the activation of the sub area 1202C, and for the time period between the activation of the sub area 1202C and the activation of the sub area 1202D.

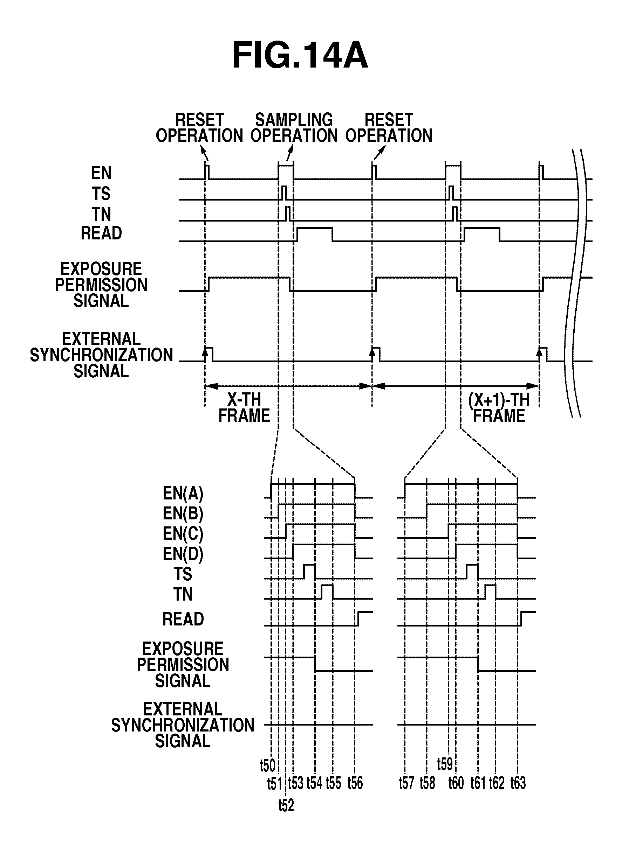

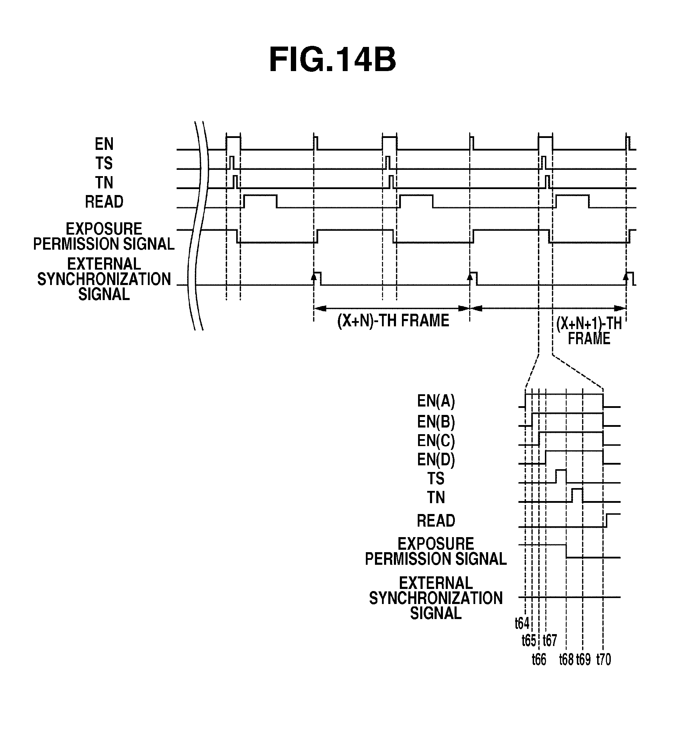

Imaging operation according to the present (third) exemplary embodiment will be described below with reference to the timing chart illustrated in Figs. 14A and 14B.

Fig. 14A is a timing chart illustrating driving signals in the X-th and (X+1)-th frames. When imaging the first to X-th frames, since the amount of light does not largely change, the signal control unit 210 sequentially turns ON the EN signals at predetermined time intervals (t50, t51, t52, and t53). An electrical signal in the X-th frame is sample-held (t54), FPN is sample-held (t55), and the EN signals are turned OFF (t56). The imaging operation will be described below based on a case where only the pixel areas 1202A and 1202B receive a large amount of light in the X-th frame image. In this case, power supply voltage drop in the sensor chip (substrate) in the pixel areas 1202A and 1202B increases. Therefore, when the EN signals are turned ON at predetermined timings, sufficient time may not be ensured until the power supply voltage of the substrate recovers.

In addition, within a substrate, the wiring resistance of the power supply line due to fine wiring causes voltage drop when a rush current flows in. As a result, since respective imaging areas have different power supply voltages at the time of sample-holding because of different total amount of light for respective pixel areas, level differences appear in boundaries between pixel areas in the image.

Accordingly, in the (X+1)-th frame, the imaging control unit 1212 changes the assert timings (t57, t58, t59, and t60) of the EN signals based on the pixel values of respective sub areas 1202 in the preceding frame to provide a stable power supply voltage at the time of sample-holding (t61 and t62).

Fig. 14B is a timing chart illustrating driving signals in the (X+N)-th and subsequent frames. In the (X+N)-th frame, when the amount of incident light for the pixel areas 1202A and 1202B decreases, it is not necessary to bring forward the timing of turning ON of the EN signals of the pixel areas 1202A and 1202B. Therefore, the imaging control unit 1212 changes the operation timings (t64, t65, t66, and t67) of the EN signals for respective pixel areas so as to perform control based on the initially set time intervals. Then, the signal output unit 211 sample-holds the light signal (t68), sample-holds the FPN signal (t69), and collectively turns OFF the EN signals (t70). Subsequently, the signal output unit 211 performs similar processing until imaging ends.

Thus, in the present exemplary embodiment, the imaging apparatus 101 determines the activation timing of the pixel circuits of respective pixel areas based on the pixel values for the preceding frame. The above-described control enables sample-holding of the signals with a stable power supply voltage in all pixel areas even if the magnitude of voltage drop changes according to the amount of incident light, restraining level differences in the image due to differences in the amount of voltage drop between respective sensor chips.

Although, in the present exemplary embodiment, the pixel amplifier activation interval is controlled, the processing is not limited thereto. The pixel amplifier activation order for each sub area 1202 may also be controlled. For example, it is possible to experimentally obtain a suitable correspondence between the average pixel value and the pixel amplifier activation order for each sub area 1202, and store relevant information in the storage unit 1204 as a look-up table. For example, it is possible to store such order information that activates the pixel amplifiers 401 earlier for a sub area 1202 having larger voltage drop.

The amplifier control unit 1203 determines the pixel amplifier activation order based on the average pixel value for each sub area 1202 calculated for each frame and on the look-up table. It is preferable that the signal control unit 210 controls the signal output unit 211 capable of independently outputting the EN(A) to EN(D) signals to sequentially assert the EN signals while delaying some EN signals according to the determined order. In this case, a fixed value is preferably used as the activation interval.

In the above-described example, the imaging control unit 1212 determines at least one of the pixel amplifier activation order and activation interval based on the representative value for the pixel values for each sub area 1202. Under the above-described control, the pixel amplifiers 401 in a sub area 1202 having larger voltage drop can be earlier activated. Accordingly, similar to the second exemplary embodiment, it becomes possible to reduce a rush current and power consumption, and improve the frame rate while restraining electromagnetic waves and noise.

A fourth exemplary embodiment combines the control based on the wiring resistance value of the power supply (according to the second exemplary embodiment) with the control based on the average pixel value for each sub area 1202 (according to the third exemplary embodiment) to calculate the priority and control the pixel amplifier activation order and activation interval for each sub area 1202. Thus, in consideration of differences in voltage drop recovery time due to differences in the wiring resistance caused by received light, and differences in the amount of voltage drop due to differences in the amount of received light, adverse effects by rush current can be suitably restrained.

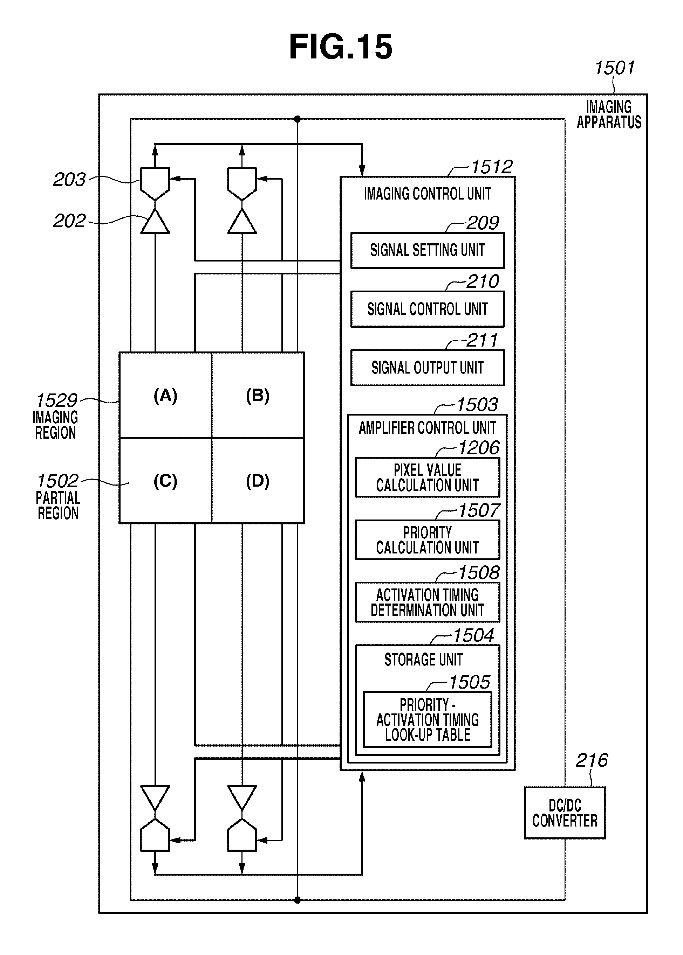

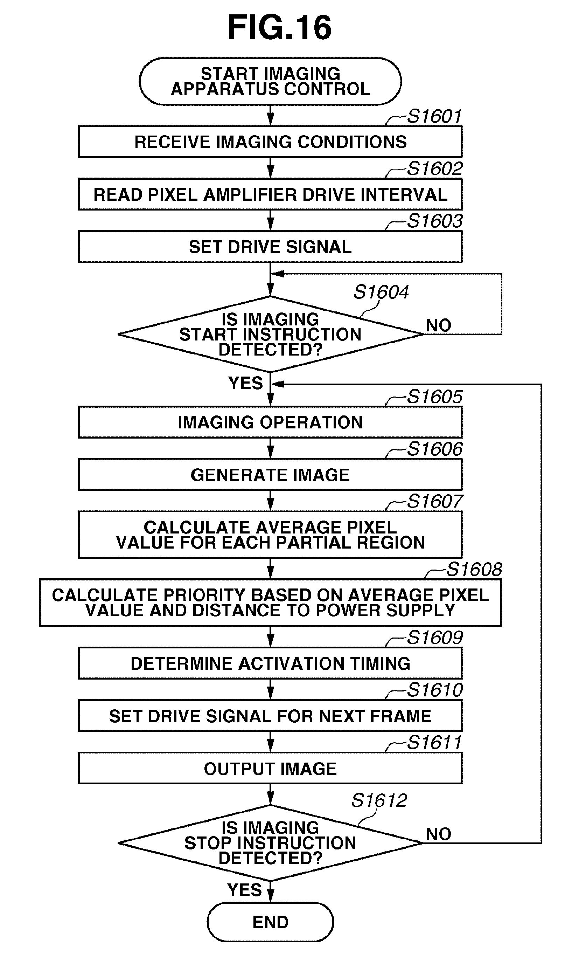

A configuration and processing of an X-ray imaging system according to the present exemplary embodiment will be described below, focusing on differences from the first to third exemplary embodiments, with reference to Figs. 15 and 16.

A configuration of an imaging apparatus 1501 will be described below with reference to Fig. 15. Fig. 15 illustrates the configuration centering on differences from the first to third exemplary embodiments. Unlike the first and second exemplary embodiments, the image sensor 111 according to the present exemplary embodiment is formed of one rectangular semiconductor substrate similar to the third exemplary embodiment. An imaging area 1529 of the image sensor 111 is divided into four sub areas 1502A, 1502B, 1502C, and 1502D for each A/D converter 203.

An amplifier control unit 1503 of an imaging control unit 1512 includes a storage unit 1504, a pixel value calculation unit 1206, a priority calculation unit 1507, and an activation timing determination unit 1508. The imaging control unit 1512 has the functions of the image generation unit 204 (not illustrated in Fig. 15) according to the first exemplary embodiment.

The storage unit 1504 stores a priority and activation timing look-up table 1505. The look-up table 1505 arranges information regarding the priority and activation timing for each sub area 1502 as table data.

The priority refers to a quantity having as variables the resistance vale of the power supply wiring and the average of pixel values for each sub area 1502. The resistance value is a parameter affecting the voltage drop recovery time and the pixel value is a parameter affecting the magnitude of voltage drop. Therefore, in consideration of these parameters, a sub area 1502 having a shorter voltage settling time has a higher priority. Generally, the larger the resistance and pixel values, the longer the voltage drop recovery time and accordingly the higher the priority. The priority may be acquired, for example, by linearly combining the resistance and the pixel value with predetermined weighting. It is preferable that an experimentally calculated value is used as a weighting factor.

Instead of using the linear combination, it is also possible to measure a voltage recovery time for two variables (resistance and pixel values) and use a look-up table in which the priority is associated with the resistance and pixel values.

The pixel value calculation unit 1206 acquires the captured image generated by the image generation unit 204, and calculates an average and total value of pixel values for each sub area 1502.

The priority calculation unit 1507 calculates the priority by using the above-described predetermined linear combination formula based on the pixel and wiring resistance values.

The activation timing determination unit 1508 determines the pixel amplifier activation interval for each sub area 1502 referring to the calculated pixel values and the look-up table 1505.

A processing flow of the imaging apparatus 1501 having the above-described configuration will be described below with reference to the flowchart illustrated in Fig. 16. As to processing similar to the first and second exemplary embodiments, descriptions will be omitted.

Processing in steps S1601 to S1606 is similar to the processing in steps S1301 to S1306 (see Fig. 13) in the third exemplary embodiment, and descriptions will be omitted.

In step S1607, the pixel value calculation unit 1206 acquires captured image data and calculates an average of pixel values for each sub area 1502.

In step S1608, the priority calculation unit 1507 calculates the priority by using the above-described predetermined linear combination formula based on the pixel values and wiring resistance values.

In step S1609, the activation timing determination unit 1508 determines the pixel amplifier activation timing for each sub area 1502 referring to the calculated pixel values and the look-up table 1505.

In step S1610, the signal setting unit 209 sets the timing of asserting the EN signals according to the determined activation interval. When the activation timing determination unit 1508 determines that the sub areas 1502A, 1502B, 1502C, and 1502D are activated in this order, the signal control unit 210 delays output by a determined time interval between the activation of the sub area 1502A and the activation of the sub area 1502B, and then instructs the signal output unit 211 to output the EN signals. The signal control unit 210 performs similar control for the time period between the activation of the sub area 1502B and the activation of the sub area 1502C, and for the time period between the activation of the sub area 1502C and the activation of the sub area 1502D.

Processing in step S1611 and subsequent steps is similar to the processing in step S1310 and subsequent steps in the third exemplary embodiment, and descriptions will be omitted.

As above-described, the fourth exemplary embodiment combines the control based on the wiring resistance value with the control based on the average pixel value for each sub area 1502 to calculate the priority and control the pixel amplifier activation order and activation interval for each sub area 1502. Thus, in consideration of differences in voltage drop recovery time due to differences in the wiring resistance caused by received light, and differences in the amount of voltage drop due to differences in the amount of received light, adverse effects by rush current can be suitably restrained.

### Other exemplary embodiments

The above-described exemplary embodiments are to be considered as illustrative and may carried out in various forms.

Although, in the above-described exemplary embodiment, each unit of the control apparatus 100 is implemented as hardware, the configuration is not limited thereto. Each unit may be implemented by executing a software program.

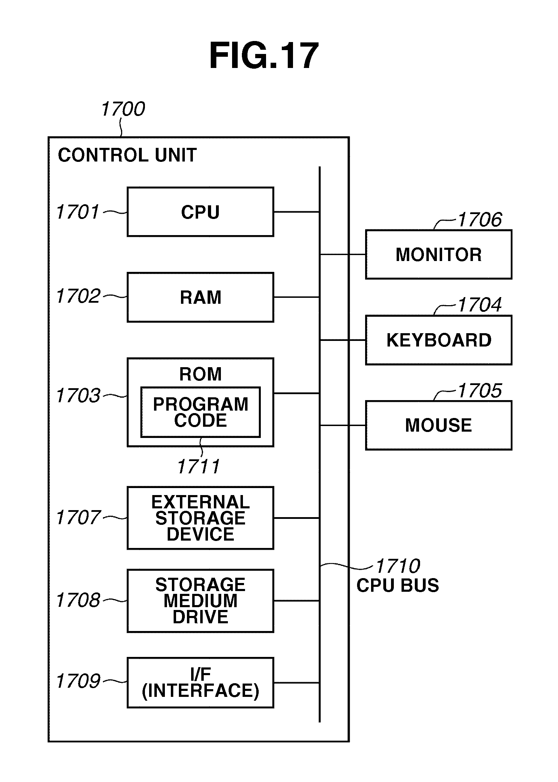

A hardware configuration of a control apparatus according to other exemplary embodiments will be described below with reference to Fig. 17.

A control apparatus 1700 includes a central processing unit (CPU) 1701, a random access memory (RAM) 1702, a read-only memory (ROM) 1703, an external storage 1707, a storage medium drive 1708, and an external communication interface (I/F) which are all connected via a bus 1710. The control apparatus 1700 is connected with a monitor 1706 (display unit) and a keyboard 1704 and a mouse 1705 (operation units). The ROM 1703 stores a program code 1711 for executing the processing illustrated in Fig. 5. When the CPU 1701 loads and executes the program code 1711, the control apparatus 1700 implements the processing illustrated in Fig. 5. When the CPU 1701 executes the program code 1711, the processing of each unit illustrated in Fig. 1 is implemented.

Also as to the imaging control unit 112 and the image generation unit 204 of the imaging apparatus 101, an apparatus having the above-described hardware configuration may achieve the processing according to the above-described exemplary embodiments. More specifically, a CPU of the apparatus may execute program codes for implementing the processing illustrated in Figs. 5, 10, 13, and 16.

Alternatively, a part of the processing of the control apparatus 100 and the imaging apparatus 101 according to the above-described exemplary embodiments may be implemented by program codes and another part of the processing may be implemented by hardware circuitry. In this case, a part of processing may be implemented by the hardware included in the control apparatus 1700 and the above-described hardware circuitry for implementing a part of each unit of the exemplary embodiments.

Program codes installed in a computer for achieving the functions of the first to fourth exemplary embodiments as described above also achieve the present invention. This means that the present invention also includes computer programs for implementing the functions and processing of the present invention. Further, computer program products such as a recording medium storing the above-described program codes are also an exemplary embodiment of the present invention.

A recording medium includes a RAM and cache data, and does not include transient media such as electric waves.

Further, the functions of the above-described exemplary embodiments can also be implemented when an operating system (OS) operating on the computer performs a part or whole of the above-described processing based on instructions of a loaded program.

The method for dividing the pixel area of the image sensor 111 is not limited to the above-described exemplary embodiments. For example, a plurality of sensor chips may be collectively handled as one pixel area. Alternatively, one sensor chip may be further divided into a plurality of small blocks, and the power of pixel circuits in respective blocks may be turned ON at different timings.

Increasing the number of divisions of the pixel area of the image sensor 111 decreases rush current, which restrains effects of unnecessary radiation due to rush current.

Further, the order and interval of turning ON of the EN signals for respective pixel areas are not limited to those according to the above-described exemplary embodiments. Although, in the above-described exemplary embodiments, the imaging control unit controls the activation timing of pixel circuits based on a fixed time period, control is not necessarily based on a fixed time interval. In this case, the imaging control unit is provided with a voltage measurement unit for measuring the power supply voltage of the sensor chip at the measuring point 218. The signal control unit 210 periodically acquires the result of measurement by the voltage measurement unit and, after confirming that the dropped power supply voltage of the sensor chip has recovered to a certain level, activates the following pixel area based on a predetermined order. This control enables both restraining image offsets and reducing unnecessary power consumption.

The sub area configuration is not limited to the above-described exemplary embodiments. Sub areas may be configured such that pixels are activated every other row or at intervals of several rows at the same timing.

The timing of drive control for turning ON the pixel circuits for each pixel area is not limited to the period of the sampling operation illustrated in Fig. 6A. When the pixel circuits need to be activated, such as in the reset operation period in Figs. 7A and 7B, by shifting the timing of turning ON of the EN signals, rush current can be reduced.

Although, in the above-described exemplary embodiments, the pixel amplifiers 401 have specifically been described, the activation timing shifting processing is applicable to elements in a pixel requiring power supply. For example, at certain timing, the switch M2 which serves as a reset circuit is turned ON to apply a voltage to the photo diode PD or the capacitance Cfd to activate the reset circuit. At another timing, the switch M5 which serves as a clamp circuit is turned ON to apply a voltage to the clamp capacitance Ccl to perform clamping. Also with respect to these elements, voltage variation can be restrained by shifting the timing of turning ON. As in the case of the above-described exemplary embodiments, by activating the reset circuit and performing clamping at the same timing, level differences in the image can be restrained.

In addition, not all pixels of the image sensor 111 need not to be subjected to the pixel amplifier activation control. For example, when an image is captured by using a part of the imaging area, the imaging control unit determines to perform the pixel amplifier activation control only on the pixels used for image generation, and does not supply the power to the pixel amplifiers 401 of other pixels. This control enables reducing of both rush current and power consumption.

Although, in the above-described exemplary embodiment, CMOS imaging devices mounted on silicon substrates are used as the image sensor 111, the type of the image sensor 111 is not limited thereto. The present invention is applicable to, for example, a PIN or MIS image sensor mounted on a amorphous silicon or polysilicon substrate as long as each pixel includes pixel amplifiers. Applying the present invention to CMOS imaging devices enables further reducing of the power consumption.

The above-described X-ray imaging apparatuses can also be used as an X-ray imaging apparatus for cardiac surgery, general medical examination, orthopedic surgery, and dental surgery.

Further, the above-described X-ray imaging apparatuses can also be used as a camera for visible light imaging in addition to X-ray imaging. When using a large sensor (having a size of about 10 cm or more), such as an X-ray sensor, since it has a large wiring resistance, the above-described activation timing change processing for each sub area is remarkably effective. Further, thermal effects resulting from power consumption can be reduced.

The above-described functions of the imaging apparatus 101 and the control apparatus 100 may be implemented by a plurality of apparatuses in a distributed manner. Alternatively, the functions of the X-ray imaging system 10 may be collectively implemented as one C arm apparatus. Further, the above-described functions of each unit of these apparatuses may be implemented by a plurality of circuits in a distributed manner, or the functions of the plurality of circuits may be implemented by one circuit.

### Other Embodiments

Aspects of the present invention can also be realized by a computer of a system or apparatus (or devices such as a CPU or MPU) that reads out and executes a program recorded on a memory device to perform the functions of the above-described embodiment (s), and by a method, the steps of which are performed by a computer of a system or apparatus by, for example, reading out and executing a program recorded on a memory device to perform the functions of the above-described embodiment(s). For this purpose, the program is provided to the computer for example via a network or from a recording medium of various types serving as the memory device (e.g., computer-readable medium).

While the present invention has been described with reference to exemplary embodiments, it is to be understood that the invention is not limited to the disclosed exemplary embodiments. The scope of the following claims is to be accorded the broadest interpretation so as to encompass all modifications, equivalent structures, and functions.

## Claims

1. An imaging apparatus (101) comprising:
an image sensor (111) having an imaging area with a plurality of pixels arranged therein, each pixel having a photoelectric conversion means and pixel amplifiers;
a control means (100) for performing control for shifting the timing of activating the pixel amplifiers for each of sub areas in the imaging area, and control for reading an electrical signal from each pixel acquired via the activated pixel amplifiers; and
a generation means for generating an image based on the read electrical signal.

2. The imaging apparatus (101) according to claim 1, wherein, when achieving a state where a predetermined number of pixel amplifiers are simultaneously been activated, the control means (100) performs control for shifting the timing of activating the predetermined number of pixel amplifiers for each of the sub areas in the imaging area, and control for reading an electrical signal from each photoelectric conversion means acquired via the activated pixel amplifiers.

3. The imaging apparatus (101) according to claim 2,
wherein the control means (100) performs control for collectively outputting the electrical signal from each pixel via the predetermined number of the activated pixel amplifiers.

4. The imaging apparatus (101) according to claim 1,
wherein the control means (100) performs control for sequentially activating the plurality of pixel amplifiers, and control for collectively outputting the electrical signal from the photoelectric conversion means, to the plurality of pixel amplifiers activated at different timings by the control for sequential activation.

5. The imaging apparatus (101) according to claim 1,
wherein the control means (100) performs control for sequentially activating the predetermined number of pixel amplifiers by shifting the activation timing for each of the sub areas in the imaging area to achieve a state where the predetermined number of pixel amplifiers are simultaneously being activated, and control for reading an electrical signal from each photoelectric conversion means acquired via the activated pixel amplifiers.

6. The imaging apparatus (101) according to claim 5,
wherein the control means (100) performs control for collectively outputting the electrical signal from each pixel via the predetermined number of the activated pixel amplifiers.

7. The imaging apparatus (101) according to claim 1,
wherein the image sensor (111) is formed of a plurality of bonded semiconductor substrates each with a plurality of pixels arranged thereon, each pixel having pixel amplifiers, and
wherein the control means (100) performs control for sequentially activating the pixel amplifiers in each of at least one semiconductor substrate, and control for reading an electrical signal from the pixel via the activated pixel amplifiers.

8. The imaging apparatus (101) according to claim 7,
wherein the control means (100) performs control for collectively outputting the electrical signal from each pixel via the predetermined number of the activated pixel amplifiers.

9. The imaging apparatus (101) according to claim 1, further comprising:
a power supply connected to the pixel amplifiers via wiring,
wherein the control means (100) controls the timing of activating the pixel amplifiers for each of the sub areas based on the resistance value of the wiring.

10. The imaging apparatus (101) according to claim 9,
wherein the control means (100) performs control for sequentially outputting a driving signal to the pixel amplifiers starting from the amplifiers for a pixel belonging to a sub area having longest wiring connection to the pixel amplifiers therein, out of the plurality of sub areas in the imaging area.

11. The imaging apparatus (101) according to claim 9,
wherein the control means (100) controls the timing of activating the pixel amplifiers for each of the sub areas according to the wiring length between the pixel amplifiers and the power supply.

12. The imaging apparatus (101) according to claim 1, further comprising:
a determination means for determining at least one of the activation order and activation interval of the pixel amplifiers in response to an instruction from an external control apparatus.

13. The imaging apparatus (101) according to claim 1, further comprising:
a determination means for determining at least one of the activation order and activation interval of the pixel amplifiers based on a representative value of pixel values for each of the sub areas.

14. The imaging apparatus (101) according to claim 13, further comprising:
a power supply connected to the pixel amplifiers via wiring,
wherein the determination means determines at least one of the activation order and activation interval of the pixel amplifiers based on a representative value of pixel values for each of the sub areas, and on the wiring resistance value between the pixel amplifiers and the power supply.

15. The imaging apparatus (101) according to any one of claims 1 to 14, wherein the control means (100) shifts the timing of outputting a signal for activating the pixel amplifiers for each of the sub areas.

16. The imaging apparatus (101) according to any one of claims 1 to 15, wherein the image sensor (111) comprises a holding means connected with the pixel amplifiers, and configured to hold an electrical signal acquired via the pixel amplifiers, and
wherein the control means (100) activates the pixel amplifiers of the pixels used for image generation and, when a predetermined time has elapsed, causes the holding means to hold the electrical signal output by the pixel amplifiers.

17. The imaging apparatus (101) according to claim 16, wherein the control means (100) performs control so that the holding means holds the electrical signal at the same timing with respect to the pixels used for image generation.

18. The imaging apparatus (101) according to claim 16, wherein the control means (100) performs control for causing, when a predetermined time has elapsed since the pixel amplifiers were activated, the holding means to hold the electrical signal acquired via the pixel amplifiers.

19. The imaging apparatus (101) according to any one of claims 1 to 18, wherein the pixel comprises:
a photoelectric conversion element;
at least one pixel amplifier;
a clamp circuit connected with the pixel amplifier;
a sample-holding means for holding the electrical signal acquired via the pixel amplifier; and
a switching means for establishing connection or perform disconnection between the pixel amplifier and a power supply means for driving the pixel amplifier.

20. An imaging apparatus (101) comprising:
an image sensor (111) comprising:
a plurality of pixels two-dimensionally arranged in an imaging area, each pixel having a photoelectric conversion element and pixel amplifiers connected to the photoelectric conversion element; and
a reading means for reading electrical signals from the plurality of pixels acquired via the pixel amplifiers;
a power supply means connected with the pixel amplifiers via wiring, and configured to supply the electric power for driving the pixel amplifiers;
an output means for outputting a driving signal for establishing connection or performing disconnection between the pixel amplifiers and the power supply; and
a control means (100) for controlling a driving signal for the image sensor (111),
wherein the control means (100) performs control for sequentially outputting a driving signal to the pixel amplifiers starting from the amplifiers for a pixel belonging to a sub area having longest wiring connection to the pixel amplifiers therein, out of the plurality of sub areas in the imaging area.

21. The imaging apparatus (101) according to any one of claims 1 to 20, wherein the image sensor (111) is an X-ray detector configured to generate an electrical signal according to a dose distribution of X-ray radiated onto the imaging apparatus (101).

22. The imaging apparatus (101) according to any one of claims 1 to 21, wherein the control means (100) performs control for collectively stopping supplying the power to the activated pixel amplifiers.

23. An imaging system comprising:
the imaging apparatus (101) according to any one of claims 1 to 22; and
a control apparatus configured to control the imaging apparatus (101).

24. A control apparatus for controlling an image sensor (111) having an imaging area with a plurality of pixels arranged therein, each pixel having pixel amplifiers, the control apparatus comprising:
a control means (100) for performing control for shifting the timing of activating the pixel amplifiers for each of the sub areas in the imaging area, and control for reading an electrical signal from each pixel acquired via the activated pixel amplifiers; and
a generation means for acquiring the read electrical signal and generate an image.

25. A method for controlling an image sensor (111) having an imaging area with a plurality of pixels arranged therein, each pixel having pixel amplifiers, the method comprising:
performing control for shifting the timing of activating the pixel amplifiers for each of the sub areas in the imaging area;
performing control for reading an electrical signal from each pixel acquired via the activated pixel amplifiers; and
generating an image based on the read electrical signal.
